# EUROPÄISCHE PATENTSCHRIFT

(11) **EP 1 289 630 B1**
(45) Veröffentlichungstag und Bekanntmachung des Hinweises auf die Patenterteilung: **05.03.2008**
(21) Anmeldenummer: 01909794.8
(22) Anmeldetag: 26.02.2001
(51) Int. Cl.: B01D 63/02, B01D 63/08, B01D 63/10

(54) **MODUL MIT MEMBRANELEMENTEN IN CROSS-FLOW UND IN DEAD-END ANORDNUNG**
MODULE WITH MEMBRANE ELEMENTS IN A CROSS-FLOW AND IN A DEAD-END ARRANGEMENT
MODULE A ELEMENTS DE MEMBRANE POUR ECOULEMENT PARTIEL ET ECOULEMENT TOTAL

(30) Priorität: 07.03.2000 DE 10011014
(43) Veröffentlichungstag der Anmeldung: 12.03.2003
(73) Patentinhaber: MAT Adsorption Technologies GmbH & Co. KG, 63784 Obernburg (DE)
(72) Erfinder: BAURMEISTER, Ulrich, 42115 Wuppertal (DE); WOLLBECK, Rudolf, 63906 Erlenbach (DE)
(74) Vertreter: Schröder, Richard
(86) Internationale Anmeldenummer: PCT/EP2001/002147
(87) Internationale Veröffentlichungsnummer: WO 2001/066237

(56) Entgegenhaltungen:
- EP-A- 0 888 809
- DE-A- 19 645 537
- US-A- 5 618 418
- US-A- 5 922 200
- US-A- 6 022 478

## Beschreibung

Die Erfindung betrifft einen Membranmodul zur stoffspezifischen Behandlung eines Fluids, enthaltend ein Gehäuse, eine Einlasseinrichtung zum Einleiten des zu behandelnden Fluids in das Gehäuse in einen Verteilerraum, eine Auslasseinrichtung zum Ableiten des behandelten Fluids aus dem Gehäuse aus einem Sammelraum, und im Gehäuse angeordnete erste Membranelemente mit poröser semipermeabler Wand sowie jeweils einem dem Verteilerraum und einem dem Sammelraum zugewandten Ende und einem durch die Wand ausgebildeten Hohlraum, wobei die ersten Membranelemente mit ihrem dem Verteilerraum zugewandten Ende in eine erste und mit ihrem dem Sammelraum zugewandten Ende in eine zweite Vergussmasse so eingebettet sind, dass die Enden durch die Vergussmassen hindurchtreten und die Hohlräume der ersten Membranelemente jeweils sowohl an dem dem Verteilerraum zugewandten Ende als auch an dem dem Sammelraum zugewandten Ende offen sind und in den Verteilerraum und in den Sammelraum mündet.

Die Erfindung betrifft des Weiteren ein Verfahren zur stoffspezifischen Behandlung eines Fluids.

Stoffspezifische Behandlungen von Fluiden gewinnen in zunehmendem Maße an Bedeutung in Anwendungsgebieten wie der Biotechnologie, der Medizin oder der chemischen Technologie. Unter Fluiden sind dabei Gase, Gasgemische sowie allgemein Flüssigkeiten wie z.B. Proteinlösungen, Suspensionen oder klare Lösungen zu subsumieren. Ein Beispiel für eine stoffspezifische Behandlung ist die Gewinnung von Wirkstoffen aus Zellsuspensionen, in denen genmodifizierte Zellen Stoffe wie Antikörper, Hormone, Wachstumsfaktoren oder Enzyme in meist kleinen Konzentrationen produziert haben. Eine wichtige Anwendung ist auch die extrakorporale Entfernung von unerwünschten Substanzen aus dem menschlichen Blutplasma sowie die Gewinnung von Komponenten wie z.B. Immunglobulinen oder Gerinnungsfaktoren aus gespendetem Blutplasma. Schließlich ist eine breite Anwendung auch die katalytische oder biokatalytische - enzymatische - Behandlung von Flüssigkeiten wie z.B. die Hydrolyse von Ölen durch Lipasen, die an einer Matrix immobilisiert sind.

Die stoffspezifische Behandlung von Fluiden erfolgt vielfach derart, dass das zu behandelnde Fluid mit einem Träger in Kontakt gebracht wird, auf und/oder in dem funktionelle Gruppen oder Substanzen immobilisiert sind, die in spezifischer, selektiver Weise mit der in dem Fluid enthaltenen Zielsubstanz, d.h. der Substanz, auf die die stoffspezifische Behandlung ausgerichtet ist, wechselwirken. Solche Wechselwirkungen können beispielsweise Kationen- oder Anionenaustausch, Hydrophil-Hydrophob-Wechselwirkung, Wasserstoffbrückenbildung, Affinität oder enzymatische oder katalytische Reaktionen und dergleichen sein. Bei der affinen Stofftrennung, wie z.B. der Affinitätschromatographie, sind an den Träger Liganden gekoppelt oder im Träger immobilisiert. Die Liganden haben die Funktion, eine einzelne Zielsubstanz oder auch eine ganze Klasse von Substanzen adsorptiv spezifisch zu binden. Diese Zielsubstanz wird als Ligat bezeichnet. Ein Beispiel für klassenspezifische Liganden sind positiv geladene Diethylaminoethyl(DEAE)-Gruppen oder negativ geladene Sulfonsäure(SO₃) - Gruppen, die die Klasse der positiv geladenen bzw. negativ geladenen Moleküle adsorbieren. Spezifische Liganden sind z.B. Antikörper gegen ein bestimmtes Protein, das als Ligat an den Antikörper gebunden wird.

Als stoffspezifische Behandlungen im Sinne der vorliegenden Erfindung werden aber auch solche Behandlungen verstanden, durch die Moleküle oder Partikel aufgrund ihrer Größe abgeschieden oder zurückgehalten werden. Für zahlreiche Anwendungen ist es wünschenswert bzw. erforderlich, ein zu behandelndes Fluid mehreren und eventuell auch unterschiedlichen stoffspezifischen Behandlungen zu unterziehen. So kann es bei Filtrationsprozessen von Suspensionen mit unterschiedlichen Partikelfraktionen erforderlich sein, zunächst größere Partikel mit einem groben offenporigen Vorfilter vorzufiltrieren und dann das Filtrat einer weiteren stoffspezifischen Behandlung z.B. nach der Größe oder nach der Affinität zu einem Liganden zu unterziehen.

Wesentliche Kriterien bei der stoffspezifischen Behandlung von Fluiden sind Produktivität und Selektivität. Mit Blick auf die Produktivität ist es wichtig, dass möglichst viele funktionelle Gruppen pro Volumeneinheit zur Verfügung stehen, die mit der in dem zu behandelnden Fluid enthaltenen Zielsubstanz in Wechselwirkung treten können. Gleichzeitig ist eine Maximierung des Transports der Zielsubstanz zu den funktionellen Gruppen bzw. Substanzen anzustreben.

In vielen derartigen Verfahren zur stoffspezifischen Behandlung von Fluiden werden heute Membranen mit poröser Struktur als Träger für funktionelle Gruppen eingesetzt. Aufgrund ihrer porösen Struktur stellen Membranen eine große innere Oberfläche zur Verfügung, so dass an die Membranen in einer hohen Konzentration pro Volumeneinheit eine große Anzahl von funktionellen Gruppen gekoppelt werden kann, die in Wechselwirkung mit den die Membran durchströmenden, zu behandelnden Fluiden treten (s. z.B. E. Klein, "Affinity Membranes", John Wiley & Sons, Inc., 1991; S. Brandt u. a., "Membrane-Based Affinity Technology for Commercial Scale Purifications", Bio/Technology Vol. 6 (1988), S. 779-782).

Über die Ausführung der verwendeten Membran kann eine Anpassung an die Erfordernisse des Behandlungsverfahrens erfolgen. Es stehen Membranen in Form von Hohlfasern oder als Flachmembranen aus unterschiedlichsten Materialien zur Verfügung, so dass eine Anpassung an die physikochemischen Eigenschaften der zu behandelnden Fluide möglich ist. Auch die Porengröße der Membranen kann so eingestellt werden, dass ein zu behandelndes Fluid z.B. mit einer in ihm enthaltenen Zielsubstanz durch die Membran konvektiv hindurchströmen kann und - im Falle der Anbindung der Zielsubstanz an die wechselwirkenden Gruppen - keine Blockierung der Membran eintritt.

Durch die Dicke der Membranwand lässt sich bei gegebener linearer Flussgeschwindigkeit die Verweilzeit des zu behandelnden Fluids in der Membran sowie der bei der Durchströmung entstehende Druckverlust beeinflussen. Aufgrund ihrer in der Regel nur geringen Wanddicke (z.B. <300 µm) zeichnen sich Membranen durch kurze Transportwege des zu behandelnden Fluids z.B. zu in den Membranen immobilisierten, wechselwirkenden Gruppen aus, wodurch die Verweilzeiten vergleichsweise kurz, die Druckverluste gering, die linearen Flussgeschwindigkeiten und damit die Bindungsraten hoch sind.

Es sind eine Reihe von solche Membranen enthaltenden Vorrichtungen beschrieben, die bei Verfahren zur stoffspezifischen Behandlung von Fluiden verwendet werden. Dabei ist zwischen dem sogenannten dead-end-Modus bzw. den dead-end-Modulen und dem cross-flow-Modus bzw. den cross-flow-Modulen zu unterscheiden.

Bei der Fahrweise im cross-flow-Modus fließt das zu behandelnde Fluid als Speisestrom parallel zur einen Seite der Membran, und ein Teil des Speisestroms tritt als Permeat durch die Membran hindurch. Daraus ergibt sich, dass bei cross-flow Modulen stets nur ein Teil der zu behandelnden Flüssigkeit, nämlich der Teil, der als Permeat durch die Membranwand tritt, der stoffspezifischen Behandlung unterzogen werden kann, die in der Regel in der Membranwand oder auch im Außenraum der Membranen erfolgt.

Beim dead-end-Modus hingegen wird das gesamte, als Speisestrom in den Membranmodul einströmende Fluid durch die Membran hindurchgeführt und auf der der Einströmseite der Membran gegenüberliegenden Abströmseite als Filtrat bzw. Permeat abgeleitet.

Die WO 90/05018 offenbart einen cross-flow Modul mit Hohlfasermembranen zum Einsatz bei Affinitätstrennverfahren. Bei diesem Modul wird eine ligathaltige Flüssigkeit über eine Einlasseinrichtung in das Modulgehäuse eingeleitet und strömt tangential entlang einer Seite der Hohlfasermembranen, in und an der die Liganden gekoppelt sind. Ein Teil der Flüssigkeit permeiert durch die Membran hindurch, wobei die Ligaten an die Liganden angelagert werden, und tritt auf der der Eintrittsseite gegenüberliegenden Membranseite als Permeatstrom aus. Über getrennte Auslasseinrichtungen werden Retentatstrom und Permeatstrom abgeleitet.

In der EP-A-0 341 413 wird ein Adsorbermodul zur Behandlung von Vollblut beschrieben, bei dem die im Modul enthaltenen, an ihren beiden Enden in Vergussmassen eingebetteten und mit Liganden versehenen Hohlfasermembranen im cross-flow-Modus lumenseitig vom Blut durchströmt werden. Hierbei tritt Plasma als Permeat durch die Hohlfasermembranwand in den die Hohlfasermembranen umschließenden Außenraum, wobei in der Membranwand die Behandlung des Plasmas erfolgt. In einer speziellen Ausführungsform besitzt dieser Modul keinen Auslass für das Permeat, sondern das als Permeat abgetrennte Plasma sammelt sich im Außenraum um die Kapillaren und tritt infolge der sich einstellenden Druckverhältnisse wieder durch die Hohlfasermembranwandung in das Lumen der Hohlfasermembran ein. Bei einem solchen Modulkonzept muss der Permeatstrom zweimal durch die Membranwand hindurchtreten, so dass der Fluss durch die Membran in einem größeren Bereich nahezu Null ist, andererseits im Eingangsbereich sehr groß ist. Ein solcher Modul hat den Nachteil, dass in den Bereichen des hohen Flusses die Bindungskapazität früh erschöpft wird, während die Bereiche geringen Flusses in ihrer Kapazität nicht ausgenutzt werden.

Die DE-A-33 02 384 beschreibt einen Hohlfasermembranen enthaltenden dead-end-Modul zur Behandlung von Blutplasma. Dieser Modul enthält zwei hintereinandergeschaltete und nebeneinander angeordnete Bündel von Hohlfasermembranen, mittels derer eine Abscheidung pathologischer Moleküle aus dem Blutplasma über Größenfraktionierung erfolgt. Die Enden der Hohlfasermembranen der beiden Membranbündel sind so in gemeinsamen Einbettungen im Gehäuse des Moduls eingegossen, dass die Hohlfasermembranen des ersten Membranbündels an ihrem dem Einlauf in den Modul zugewandten Ende offen und an ihrem entgegengesetzten Ende verschlossen sind, wohingegen die Hohlfasermembranen des zweiten Membranbündels an ihrem dem Auslauf aus dem Modul zugewandten Ende geöffnet und an ihrem anderen Ende verschlossen sind. Die offenen Enden der beiden Hohlfaserbündel sind also entgegengesetzt angeordnet. Im Betrieb strömt das zu behandelnde Blutplasma, aus dem die pathogenen Bestandteile abgefiltert werden sollen, im dead-end-Modus zunächst über die geöffneten Enden der Hohlfasermembranen des ersten Membranbündels in das Lumen dieser Membranen und durch deren Wand in den extraluminaren Bereich. Das somit einmal filtrierte Plasma fließt dann von außen nach innen in das Lumen der Hohlfasermembranen des zweiten Membranbündels und verlässt diese durch deren geöffnete Enden.

In der DE-A-37 09 432 wird ein dead-end Modul zur Sterilisation von flüssigen Medien offenbart, der zwei hintereinandergeschaltete, nebeneinander angeordnete Bündel von Hohlfasermembranen enthält und von seinem Aufbau her dem in der DE-A-33 02 384 beschriebenen ähnlich ist. Bei dem Modul gemäß DE-A-37 09 432 kann die Membran mindestens eines der Hohlfaserbündel Adsorptivstoffe aufweisen. Die Bündel können darüber hinaus von einer zusätzlichen Filtrationseinrichtung in Form eines semipermeablen Schlauchs umschlossen sein, wobei diese ebenfalls Adsorptivstoffe aufweisen kann.

Die US 5,693,230 offenbart ebenfalls einen dead-end Modul mit zwei Gruppen von Hohlfasermembranen, wobei alle Hohlfasermembranen an ihrem einem Ende offen und an ihrem anderen Ende verschlossen sind. Hierbei enthält der Modul erste Hohlfasermembranen zur Zuführung des zu behandelnden Fluids und zweite Hohlfasermembranen zur Abführung des behandelten Fluids. Das zu behandelnde Fluid strömt durch die offenen Enden der ersten Hohlfasermembranen in deren Lumen ein, tritt durch die poröse Wand dieser Membranen hindurch in den Außenraum um die ersten und zweiten Hohlfasermembranen und wird von dort durch die poröse Wand der zweiten Hohlfasermembranen in deren Lumen eingeleitet und durch deren offene Enden abgeleitet. Im Außenraum wird das Fluid mit einem sich dort befindenden Behandlungsmedium in Kontakt gebracht und behandelt.

Gemäß der US-A-6022 478 wird ein Modul eingesetzt, in dem mehrere Membranen nacheinander gleich zeitig um-und durchsfrömt werden.

Generell ist im Unterschied zu den cross-flow Modulen ein Nachteil von dead-end Modulen, dass sie für die Behandlung von Suspensionen nur in stark eingeschränktem Maße geeignet sind, da bei dieser Konstruktion in der Regel die suspendierten Partikel von der eingesetzten Membran zurückgehalten werden. Darüber hinaus sind dead-end Membranmodule nach dem Stand der Technik insofern wenig flexibel einsetzbar, da bei ihrer Anwendung in der Regel nur eine einzige stoffspezifische Behandlung an einem zu behandelnden Fluid durchgeführt werden kann.

Es ist daher Aufgabe der vorliegenden Erfindung, einen Membranmodul der eingangs genannten Art zur Verfügung zu stellen, bei welchem die Nachteile der Membranmodule des Stands der Technik zumindest reduziert sind und der für die stoffspezifische Behandlung sowohl von klaren Lösungen als auch insbesondere von Suspensionen geeignet ist, flexibel an die jeweilige Fluidbehandlung angepasst werden kann und mittels dessen insbesondere verschiedenartige aufeinanderfolgende stoffspezifische Behandlungen ermöglicht werden.

Es ist des Weiteren Aufgabe der vorliegenden Erfindung, ein Verfahren zur effizienten stoffspezifischen Behandlung von Fluiden unter Verwendung eines semipermeable Membranen mit poröser Struktur enthaltenden Moduls bereitzustellen, bei dem die oben genannten Nachteile zumindest reduziert sind und z.B. eine bessere Ausnutzung von im Modul immobilisierten funktionellen Gruppen und die stoffspezifische Behandlung von Suspensionen ermöglicht wird.

Die Aufgabe wird bei einem Membranmodul gemäß Oberbegriff des Anspruchs 1 dadurch gelöst, dass der Membranmodul des Weiteren im Gehäuse angeordnete zweite Membranelemente mit poröser semipermeabler Wand sowie jeweils einem dem Verteilerraum und einem dem Sammelraum zugewandten Ende und einem durch die Wand ausgebildeten Hohlraum enthält, wobei die zweiten Membranelemente mit ihrem dem Sammelraum zugewandten Ende zusammen mit den ersten Membranelementen in die zweite Vergussmasse so eingebettet sind, dass diese Enden durch die zweite Vergussmasse hindurchtreten und die Hohlräume der zweiten Membranelemente an diesem Ende offen sind und in den Sammelraum münden, und die zweiten Membranelemente an ihren dem Verteilerraum zugewandten Enden geschlossen sind, wobei die ersten und die zweiten Membranelemente eine Membranelementeanordnung ausbilden und wobei zwischen den Vergussmassen ein gegenüber dem Verteilerraum und gegenüber dem Sammelraum fluiddicht abgetrennter und durch die Vergussmassen, die Innenwand des Gehäuses und die Membranelemente begrenzter Aussenraum ausgebildet ist.

Hierbei ist die Erstreckung der ersten und zweiten Membranelemente in der Richtung zwischen der dem Verteilerraum zugewandten Vergussmasse und der dem Sammelraum zugewandten Vergussmasse größer als diejenige in mindestens einer der dazu senkrechten Richtungen.

Unter einer fluiddichten Abtrennung des Außenraums gegenüber dem Verteilerraum bzw. dem Sammelraum wird hier verstanden, dass ein Fluid, welches sich im Verteilerraum befindet, nur über die ersten Membranelemente in den Außenraum gelangen kann und umgekehrt und dass ein Fluid, welches sich im Außenraum befindet, nur über die ersten bzw. die zweiten Membranelemente in den Sammelraum gelangen kann und umgekehrt.

In der Anwendung wird das zu behandelnde Fluid als Speisestrom in den erfindungsgemäßen Membranmodul eingeleitet und entlang der dem Hohlraum zugewandten Seite der Wände der ersten Membranelemente im cross-flow-Modus so vorbeigeführt, dass ein Teil des Speisestroms durch die semipermeablen Wände der ersten Membranelemente als Permeat hindurchtritt und der Rest des Speisestroms als Retentat durch die Hohlräume der ersten Membranelemente strömt und schließlich aus diesen an ihrem dem Sammelraum zugewandten Ende austritt. Das Permeat durchströmt nach Austritt aus den Wänden der ersten Membranelemente den Außenraum im wesentlichen in Richtung auf die zweiten Membranelemente. Zumindest ein wesentlicher Teil des Permeats strömt im dead-end-Modus durch die semipermeablen Wände der zweiten Membranelemente in deren Hohlräume ein, durchströmt diese Hohlräume und tritt aus den zweiten Membranelementen an ihrem geöffneten, dem Sammelraum zugewandten Ende in den Sammelraum aus. Dort wird es mit dem aus den ersten Membranelementen austretenden Retentat vereinigt. Zwischen der Abtrennung vom Speisestrom und der Vereinigung mit dem Retentat wird das Permeat mindestens einer stoffspezifischen Behandlung unterworfen.

Je nach Ausführungsform des erfindungsgemäßen Membranmoduls, d.h. abhängig von den im Modul herrschenden Druckverhältnissen, ist es möglich, dass ein Teil des in den Außenraum eingeströmten Permeats nicht durch die zweiten Membranelemente strömt, sondern über eine Rückfiltration im Bereich vor der dem Sammelraum zugewandten Vergussmasse durch die Wände der ersten Membranelemente in deren Hohlraum zurückströmt, um sich dort mit dem Retentat zu vereinigen. Erfindungsgemäß strömt jedoch zumindest ein wesentlicher Teil des Permeats und bevorzugt zumindest 50 % des Permeats durch die zweiten Membranelemente.

Der aus Retentat und Permeat vereinigte Fluidstrom verlässt das Gehäuse als behandeltes Fluid über die Auslasseinrichtung. In Gestalt der ersten und der zweiten Membranelemente sind im erfindungsgemäßen Membranmodul mindestens zwei in Reihe geschaltete unterschiedliche Behandlungsstufen integriert, wobei für die im dead-end-Modus durchströmte Behandlungsstufe keine separaten Pumpen oder Regelorgane erforderlich sind. Die die Behandlungsstufen verlassenden Teilströme werden innerhalb des Moduls wieder zusammengeführt und verlassen als gemeinsamer Fluidstrom den erfindungsgemäßen Membranmodul.

Die Aufgabe wird daher des Weiteren durch ein Verfahren zur stoffspezifischen Behandlung eines Fluids gelöst, welches zumindest die Schritte umfasst:
a) Einleiten des zu behandelnden Fluids als Speisestrom in einen mindestens eine erste Membran und mindestens eine zweite Membran enthaltenden Modul,
b) Vorbeileiten des Speisestroms im cross-flow-Modus an der einen Seite der ersten Membran, so dass ein Teil des Speisestroms als Permeat durch die erste Membran hindurchtritt und auf der anderen Seite der ersten Membran aus dieser austritt, während der Rest des Speisestroms als Retentat entlang der ersten Membran weiterströmt,
c) Einleiten zumindest eines wesentlichen Teils des Permeats im dead-end-Modus in die zweite Membran auf der einen Seite dieser Membran, Durchströmen der zweiten Membran und Ausleiten aus der zweiten Membran auf deren anderer Seite,
d) Vereinigung des Permeats mit dem Retentat zum behandelten Fluid innerhalb des Moduls und
e) Ableiten des behandelten Fluids aus dem Modul,
wobei das Permeat zwischen der Abtrennung vom Speisestrom und der Vereinigung mit dem Retentat mindestens einer stoffspezifischen Behandlung unterworfen wird.

Aufgrund der speziellen Kombination von im cross-flow-Modus mit im dead-end-Modus an- bzw. durchströmten Membranen lässt sich das erfindungsgemäße Verfahren vorteilhaft zur Behandlung von Suspensionen einsetzen. In einer bevorzugten Ausgestaltung wird bei dem erfindungsgemäßen Verfahren ein erfindungsgemäßer Membranmodul eingesetzt.

Erfindungsgemäß sind die ersten sowie die zweiten Membranelemente mit ihren dem Sammelraum zugewandten, offenen Enden in der an den Sammelraum angrenzenden zweiten Vergussmasse eingebettet. Hierbei treten sie mit diesem Ende durch diese Vergussmasse hindurch, so dass eine Öffnung des jeweiligen Hohlraums zum Sammelraum gegeben ist. Die dem Verteilerraum zugewandten offenen Enden der ersten Membranelemente sind erfindungsgemäß in der an den Verteilerraum angrenzenden ersten Vergussmasse so eingebettet, dass sie mit diesen Enden durch diese Vergussmasse hindurchtreten und so eine Öffnung der Hohlräume der ersten Membranelemente auch zum Verteilerraum hin gegeben ist.

Als Vergussmassen kommen üblicherweise für diesen Zweck eingesetzte Materialien wie z.B. Epoxydharze, Polyurethanharze oder auch Schmelzkleber in Frage, die im flüssigen Zustand eine einfache und fluiddichte Einbettung der Membranelemente erlauben. Unter Vergussmassen sind jedoch auch solche Ausführungsformen zu verstehen, bei denen eine Vergussmasse zunächst vorgefertigt worden ist, z.B. als ein mit entsprechenden Aussparungen zur Aufnahme der Membranelemente versehenes Spritzgussteil, in das die Membranelemente beispielsweise mittels eines Klebers fluiddicht eingeklebt werden.

Die geschlossenen Enden der zweiten Membranelemente können bereits im Außenraum um die Membranelemente enden und somit nicht eingebettet sein. Sie können jedoch auch zusammen mit den dem Verteilerraum zugewandten Enden der ersten Membranelemente in die an den Verteilerraum angrenzende erste Vergussmasse eingebettet sein, dann jedoch in der Weise, dass die dem Verteilerraum zugewandten Enden der zweiten Membranelemente geschlossen bleiben.

In einer bevorzugten Ausgestaltung des erfindungsgemäßen Membranmoduls ist die Membranelementeanordnung aus flächigen Schichten aufgebaut. Hierdurch lässt sich ein hoher Grad an Ordnung erreichen und eine definierte Strömung zwischen den ersten und den zweiten Membranelementen erreichen. Dies gilt insbesondere dann, wenn erste und zweite Membranelemente direkt zueinander benachbart sind und beispielsweise in einer alternierenden Reihenfolge zueinander angeordnet sind. Besonders bevorzugt ist ein Aufbau der Membranelementeanordnung aus flächigen Schichten, die zu einem Stapel aufeinandergelegt sind. In einer weiteren besonders bevorzugten Ausführungsform besteht die Membranelementeanordnung aus flächigen Schichten, die spiralförmig um eine Wickelachse gewickelt sind. Unter einer flächigen Schicht im Sinne der vorliegenden Erfindung wird eine solche Schicht verstanden, deren Abmessungen im ebenen Zustand, d.h. also z.B. auch vor einem spiralförmigen Aufwickeln, in Erstreckungsrichtung der Membranelemente zwischen ihren Enden sowie in einer Richtung quer dazu wesentlich größer ist als in der verbleibenden zweiten Richtung quer zu den Membranelementen, die die Dicke der Schicht bestimmt.

Vorzugsweise umfasst die Membranelementeanordnung erste Schichten und zweite Schichten, wobei die ersten Schichten nur erste Membranelemente und die zweiten Schichten nur zweite Membranelemente enthalten. Besonders bevorzugt umfasst die Anordnung eine alternierende Abfolge von ersten und zweiten Schichten.

In einer bevorzugten Ausführungsform der Erfindung handelt es sich bei den ersten und den zweiten Membranelementen um Hohlfasermembranen, und die Hohlräume der Membranelemente werden durch die Lumina der Hohlfasermembranen ausgebildet. Bei der bevorzugten Ausführungsform eines Aufbaus der Membranelementeanordnung in flächigen Schichten sind die Hohlfasermembranen innerhalb der Schichten zueinander im wesentlichen parallel zueinander angeordnet. Die Hohlfasermembranen sind so in das Gehäuse des Membranmoduls eingebettet, dass die Lumina der als erste Membranelemente fungierenden ersten Hohlfasermembranen an deren beiden Enden geöffnet sind und die Lumina der als zweite Membranelemente dienenden zweiten Hohlfasermembranen nur an deren dem Sammelraum zugewandten Ende geöffnet sind und an deren dem Verteilerraum zugewandten Ende geschlossen sind. Der Verschluss an dem geschlossenen Ende der zweiten Hohlfasermembranen kann z.B. durch die Vergussmasse oder durch ein Abschweißen bewirkt werden. Gemäß einer bevorzugten Ausführungsform sind zur Ausbildung der zweiten Membranelemente entsprechend in ihrer Länge bemessene Hohlfasern so U-förmig gebogen oder geknickt bzw. schlaufenförmig abgelegt, dass sich zwei miteinander verbundene zweite Membranelemente ergeben, deren Hohlraum in Richtung des Verteilerraums geschlossen und in Richtung des Sammelraums geöffnet ist.

Anstelle von Hohlfasermembranen können auch rohrförmige Membranen oder schlauchförmige Membranen eingesetzt werden, deren Durchmesser und Wandstärken in der Regel größer sind als die der Hohlfasermembranen.

Es können Hohlfasermembranen mit verschiedenen äußeren Konturen, d.h. mit im Querschnitt betrachtet verschiedenen äußeren Umrissen eingesetzt werden. Die Hohlfasermembranen können beispielsweise eine im wesentlichen runde bzw. kreisförmige, dreieckige, viereckige, sechseckige oder achteckige Kontur aufweisen, sie können auch oval, elliptisch, dreilappig, vierlappig usw. ausgebildet sein. Für den Einsatz im erfindungsgemäßen Membranmodul haben sich Hohlfasermembranen bewährt, die eine Wandstärke zwischen 15 und 900 µm aufweisen, bestens bewährt haben sich Hohlfasermembranen mit einer Wandstärke zwischen 30 bis 100 µm für die ersten Membranelemente und zwischen 100 und 300 µm für die zweiten Membranelemente.

Vorzugsweise beträgt der hydraulische Durchmesser des Lumens der eingesetzten Hohlfasermembranen 50 bis 1500 µm, besonders bevorzugt sind Hohlfasermembranen mit einem hydraulischen Durchmesser des Lumens zwischen 80 und 300 µm mit der Definition des hydraulischen Durchmessers als 4*A/U, wobei A die Fläche des Strömungsquerschnitts des Hohlfaserlumens und U den Umfang des Strömungsquerschnitts des jeweiligen Hohlfaserlumens bezeichnet. Je nach Anwendung und insbesondere bei unterschiedlichen Aufgaben der ersten und zweiten Membranelemente können die hydraulischen Durchmesser der als erste und der als zweite Membranelemente eingesetzten Hohlfasermembranen unterschiedlich groß gewählt werden.

Die ersten und zweiten Hohlfasermembranen sind bevorzugt in jeweils mindestens eine Hohlfasermatte eingebunden, wobei die Hohlfasermembranen innerhalb einer Matte im wesentlichen parallel zueinander angeordnet sind. In diese Hohlfasermatten sind die Hohlfasermembranen bevorzugt mittels textiler Fäden eingebunden. Derartige Matten lassen sich vorteilhaft nach bekannten Verfahren als Wirkmatte oder Webmatte, aber auch als Webbändchen, Strick- oder als Häkelmatte herstellen. In den Fällen des Webens oder Wirkens sind die textilen Fäden die quer zu den Hohlfasermembranen verlaufenden Web- bzw. Kettfäden. Durch diese Querfäden werden die Hohlfasermembranen in zueinander im wesentlichen paralleler Anordnung gehalten. Mittels derartiger Hohlfasermatten lässt sich auf einfache Weise ein Aufbau der Membranelementeanordnung aus flächigen Schichten realisieren.

In einer bevorzugten Ausführungsform enthält eine solche Hohlfasermatte sowohl erste als auch zweite Hohlfasermembranen. Innerhalb solcher Matten können die ersten und zweiten Hohlfasermembranen je nach den Anforderungen an den erfindungsgemäßen Membranmodul in alternierender Reihenfolge angeordnet sein oder auch in unterschiedlicher Anzahl, wobei dann die ersten Hohlfasermembranen und die zweiten Hohlfasermembranen auch in Gruppen zusammengefasst sein können.

Hohlfasermatten mit ersten und zweiten Hohlfasermembranen in alternierender Reihenfolge lassen sich z.B. durch gemeinsames Verwirken von abwechselnd parallel als Schussfaden in eine Raschelmaschine zugeführten Hohlfasermembranen herstellen. Dabei werden die ersten von den zweiten Hohlfasermembranen vor der gemeinsamen Einbettung in die Vergussmasse dadurch unterschieden, dass die ersten Hohlfasermembranen z.B. durch eine mäanderförmige Verlegung auf beiden Enden der Matte verschlossen werden, während die zweiten Hohlfasermembranen innerhalb einer Matte an einer Kante der Matte geöffnet werden. Die offene Seite wird bei der Einbettung in eine Vergussmasse durch die Vergussmasse verschlossen. In die zuvor geschlossenen Enden kann hingegen bei der Einbettung keine Vergussmasse eindringen, so dass bei dem anschließenden Schnitt durch die Vergussmasse die anfangs verschlossenen Lumina der Hohlfasermembranen an dieser Seite geöffnet werden.

Eine solche Matte kann aber z.B. auch auf einer Raschelmaschine mit mäanderförmiger Verlegung der Hohlfäden als Schussfäden so hergestellt werden, dass die Schlaufen der zweiten Hohlfasermembranen an der einen Kante der Matte soweit vom Rand der Matte weg in die Matte verlegt werden, dass der Schnitt nach dem Einbetten in die Vergussmasse die zweiten Kapillarmembranen auf dieser Seite nicht erfasst und somit die geschlossene Seite der zweiten Membranelemente entsteht. Die übrigen Schlaufen der ersten und zweiten Membranelemente werden beim Rascheln so gelegt, dass sie nach dem Einbetten in die Vergussmasse weggeschnitten und damit die Lumina aller Hohlfasermembranen auf dieser Seite geöffnet werden.

Unterschiedliche Flächen- und Massenverhältnisse können in einer Matte durch eine unterschiedliche Zahl der sich abwechselnden ersten und zweiten Hohlfasermembranen realisiert werden. Bei Einbindung von ersten und zweiten Hohlfasermembranen innerhalb derselben Hohlfasermatte ist es vorteilhaft, wenn erste und zweite Hohlfasermembranen einen zumindest nahezu gleichen Außendurchmesser aufweisen.

Gemäß einer weiteren bevorzugten Ausführungsform enthält jede Hohlfasermatte nur erste Hohlfasermembranen oder nur zweite Hohlfasermembranen. Matten mit ersten Hohlfasermembranen lassen sich z.B. aus mäanderförmig gewebten oder gewirkten Hohlfasermembranmatten herstellen, an deren Seitenkanten die Hohlfasermembranen im Bogen verlaufen und die daher an diesen Kanten zunächst geschlossen sind. Nach der Einbettung können die Hohlfasermembranen bei Beschneidung der jeweiligen Vergussmasse durch Abschneiden der Endbögen an beiden Kante der Matte beidseitig geöffnet werden. Entsprechend können Matten mit zweiten Hohlfasermembranen aus solchen mäanderförmig gewebten oder gewirkten Hohlfasermembranmatten hergestellt werden, indem nur an einer der beiden Kanten die Endbögen abgeschnitten und so die Hohlfasermembranen einseitig geöffnet werden. Vorzugsweise wird jedoch zur Herstellung einer Matte mit zweiten Hohlfasermembranen eine solche mäanderförmig gewebte oder gewirkte Matte mittig geteilt, so dass zwei Hälften mit Hohlfasermembranen entstehen, deren Hohlräume einseitig geöffnet sind.

Gemäß einer weiteren Ausgestaltung der Erfindung sind die Hohlfasermembranen mittels flächiger, vorzugsweise streifenförmiger Verbindungselemente in die jeweilige Hohlfasermatte eingebunden. Solche streifenförmigen Verbindungselemente können quer aber auch unter einem anderen Winkel zu den zueinander parallelen Hohlfasermembranen verlaufen und beispielsweise mittels eines punktförmig aufgetragenen Klebers z.B. auf Polyurethanbasis auf diese auflaminiert sein.

In einer vorteilhaften Ausführungsform sind mehrere Hohlfasermatten zu einem Stapel aufeinandergelegt, wobei die Hohlfasermatten ebene flächige Schichten ausbilden. In einer weiteren bevorzugten Ausführung der erfindungsgemäßen Vorrichtung ist mindestens eine erste und zweite Hohlfasermembranen enthaltende Hohlfasermatte zick-zack-förmig zu einem Stapel gefaltet. Gemäß einer weiteren bevorzugten Ausführungsform sind mindestens eine nur erste Hohlfasermembranen enthaltende und mindestens eine nur zweite Hohlfasermembranen enthaltende Hohlfasermatte übereinandergelegt und miteinander zick-zack-förmig zu einem Stapel gefaltet.

In einer ebenfalls vorteilhaften Ausführungsform ist mindestens eine erste und zweite Hohlfasermembranen enthaltende Hohlfasermatte spiralförmig um eine zu den Hohlfasermembranen parallele Wickelachse gewickelt, so dass sich ein Aufbau aus flächigen Schichten ergibt. Bei Verwendung von Hohlfasermatten, die nur erste bzw. nur zweite Hohlfasermembranen enthalten, sind mindestens eine nur erste Hohlfasermembranen enthaltende und mindestens eine nur zweite Hohlfasermembranen enthaltende Hohlfasermatte übereinandergelegt und miteinander spiralförmig um eine zu den Hohlfasermembranen parallele Wickelachse gewickelt. Bei der Wickelachse kann es sich beispielsweise auch um einen Wickelkern mit beliebigem Querschnitt und beliebiger Struktur handeln. Bevorzugt wird ein ziehbarer Wickelkern, der nach dem Wickeln herausgezogen werden kann, ohne einen wesentlichen Leerraum in dem nach dem Wickeln entstandenen Bündel zu hinterlassen.

In einer bevorzugten Ausgestaltung des erfindungsgemäßen Membranmoduls sind die Hohlfasermembranen innerhalb der flächigen Schichten und diejenigen benachbarter Schichten zueinander im wesentlichen parallel. Je nach Anwendung der erfindungsgemäßen Membranmodule können aber auch definierte Zwischenräume zwischen den Hohlfasermembranen, d.h. ist eine definierte Ausgestaltung des Außenraums erforderlich sein. In diesen Fällen ist eine Membranelementeanordnung von Vorteil, die durch spiralförmiges Wickeln von mindestens zwei übereinandergelegten Hohlfasermatten um eine Achse oder einen Kern hergestellt worden ist, wobei die Hohlfasermembranen innerhalb jeder Matte in einem gegenseitigen Abstand zueinander angeordnet sind und wobei die Hohlfasermatten so übereinandergelegt sind, dass die Hohlfäden der übereinandergelegten Hohlfasermatten in eine sich überkreuzende Anordnung gebracht sind. Die Herstellung derartiger Bündel wird eingehend in der EP-A-0 285 812 beschrieben. Für solche Bündel kann der sich zwischen den überkreuzt angeordneten Hohlfasermembranen ausbildende Winkel zwischen 0° und 120° liegen, als günstig haben sich Winkel zwischen 30° und 90° herausgestellt.

In einer weiteren bevorzugten Ausgestaltung des erfindungsgemäßen Membranmoduls sind die ersten Membranelemente Hohlfasermembranen und die zweiten Membranelemente jeweils aus mindestens einer Flachmembran ausgebildet. Ein derartiges zweites Membranelement aus mindestens einer Flachmembran besteht entsprechend einer vorteilhaften Ausführungsform aus einem U-förmig gefalteten, im wesentlichen rechteckigen oder quadratischen Stück einer Flachmembran, wobei neben der Faltkante zwei weitere Seitenkanten, also z.B. die zur Faltkante senkrechten Seitenkanten beispielsweise durch Verschweißen oder Verkleben geschlossen sind. Die bei der U-förmigen Faltung eines solchen Stücks einer Flachmembran entstehenden zwei Schenkel werden mittels Abstandshalter auf Abstand gehalten und bilden so zusammen mit der geschlossenen Faltkante und den geschlossenen Seitenkanten einen an einer Seite geöffneten Hohlraum aus. Die geöffnete Seitenkante ist im erfindungsgemäßen Membranmodul in Richtung des Sammelraums angeordnet.

Gemäß einer weiteren vorteilhaften Ausgestaltung der Erfindung bestehen derartige aus mindestens einer Flachmembran ausgebildete zweite Membranelemente aus jeweils zwei gleich großen, zueinander im wesentlichen parallelen und im wesentlichen rechteckigen oder quadratischen Flachmembranen, die so zueinander angeordnet sind, dass ihre Kanten parallel zueinander verlaufen, wobei die Flachmembranen gegeneinander durch Abstandshalter auf Abstand gehalten werden. Die beiden Flachmembranen sind an drei Kanten, d.h. an ihrer in Richtung Verteilerraum weisenden Kante sowie den dazu senkrechten Kanten formschlüssig miteinander verbunden, beispielsweise miteinander verschweißt oder verklebt, so dass durch die Flachmembranen die Schenkel des Membranelements ausgebildet werden. Der Hohlraum wird wiederum durch die Schenkel und die formschlüssig verbundenen Kanten ausgebildet. Die verbleibende offene Seitenkante ist in Richtung des Sammelraums angeordnet.

Die beiden eingesetzten Flachmembranstücke können gleich sein, sie können jedoch auch unterschiedlich sein z.B. im Hinblick auf ihr Material, ihre Struktur oder die zur Wechselwirkung mit Zielsubstanzen vorgesehenen funktionellen Gruppen. Auch kann eine der beiden Flachmembranen gegen eine beispielsweise fluidundurchlässige Folie eingetauscht werden, wo dies - etwa aus Gründen der Stabilität der Membranelemente oder aus Fertigungsgesichtspunkten - günstig erscheint.

Die beschriebenen Flachmembranelemente sind im erfindungsgemäßen Membranmodul so angeordnet, dass die verbleibende offene Seitenkante in Richtung des Sammelraums weist und der Hohlraum zu diesem geöffnet ist.

Die Abstandshalter, die, wie beschrieben, für einen definierten Abstand zwischen den Schenkeln der aus Flachmembranen ausgebildeten Behandlungselemente sorgen und gleichzeitig fluiddurchlässig sein sollen, können als separate Elemente ausgebildet sein. Als Abstandshalter kann ein für Fluide durchlässiges Material wie z.B. ein Vlies oder ein Gewebe eingesetzt werden. Die Abstandshalterfunktion auf der Innenseite der Membran kann jedoch auch in die Membran bzw. die Folie selbst integriert werden z.B. durch rillenförmige, noppenförmige oder andere profilierte Oberflächenstrukturen.

Die in der erfindungsgemäßen Vorrichtung bzw. zur Durchführung des erfindungsgemäßen Verfahrens eingesetzten Flachmembranen weisen vorzugsweise eine Wandstärke zwischen 15 µm und 500 µm auf, besonders bevorzugt sind Flachmembranen mit einer Wandstärke zwischen 100 µm und 300 µm.

Solchermaßen aus Flachmembranen ausgebildete zweite Membranelemente können in ebener Form eingesetzt werden und mit vorzugsweise in Hohlfasermatten eingebundenen ersten Hohlfasern zu Stapeln übereinander angeordnet werden, die dann aus ersten, die Hohlfasermembranelemente enthaltenden, und aus zweiten, die Flachmembranelemente enthaltenden ebenen flächigen Schichten aufgebaut sind. Auch eine zick-zack-förmige Faltung zusammen mit erste Hohlfasermembranen enthaltenden Hohlfasermatten lässt sich auf vorteilhafte Weise realisieren. Des Weiteren lassen sich die aus Flachmembranen aufgebauten zweiten Membranelemente auch zusammen mit ersten Membranelementen in Form von Hohlfasermembranen spiralförmig um eine Wickelachse parallel zu den Hohlfasermembranen und senkrecht zur geöffneten Kante der zweiten Membranelemente aufwickeln, wobei auch hier die Hohlfasermembranen die ersten und die Flachmembranen die zweiten Schichten ausbilden. Bei den genannten Anordnungen können die zweiten Schichten auch jeweils aus einem einzelnen Flachmembranelement bestehen.

Bei der Durchströmung der Wände der ersten Membranelemente, bei der Durchströmung des Außenraums und/oder bei der Durchströmung der zweiten Membranelemente wird der als Permeatstrom abgetrennte Teil des zu behandelnden Fluids einer stoffspezifischen Behandlung unterzogen. Hierbei kann bei der Durchströmung der Wände der ersten Membranelemente und/oder bei der Durchströmung der zweiten Membranelemente die stoffspezifische Fluidbehandlung allein eine Fraktionierung hinsichtlich im Fluid enthaltener Substanzen aufgrund ihrer Größe sein, wobei solche Substanzen z.B. in Form von Molekülen oder Partikeln vorliegen können. Vorzugsweise ist aber im Gehäuse des erfindungsgemäßen Membranmoduls eine Matrix enthalten, auf und/oder in der funktionelle Gruppen immobilisiert sind, die in spezifischer, selektiver Weise mit einer einzelnen im zu behandelnden Fluid enthaltenen Zielsubstanz oder mehreren Zielsubstanzen, d.h. Substanzen, auf die die stoffspezifische Behandlung ausgerichtet ist, wechselwirken.

In einer bevorzugten Ausgestaltung der Erfindung sind die ersten Membranelemente und/oder zweiten Membranelemente die Matrix für die funktionellen Gruppen, und die funktionellen Gruppen sind auf und/oder in den Membranelementen immobilisiert. Hierbei sind besonders bevorzugt sowohl die ersten als auch die zweiten Membranelemente Matrix für funktionelle Gruppen. Es ist jedoch auch möglich, dass nur auf und/oder in den ersten oder auf und/oder in den zweiten Membranelementen funktionelle Gruppen immobilisiert sind. Für den Fall, dass sowohl die ersten als auch die zweiten Membranelemente Matrix für funktionelle Gruppen sind, können die funktionellen Gruppen in den jeweiligen Membranelementen gleich oder unterschiedlich sein.

Bei einer ebenfalls bevorzugten Ausführungsform der erfindungsgemäßen Vorrichtung sind zwischen die ersten und zweiten Membranelemente fluiddurchlässige, d. h. für das zu behandelnde Fluid durchlässige Trägermaterialien eingebracht, die Matrix für die funktionellen Gruppen sind, d.h. auf und/oder in diesen als Matrix fungierenden Trägermaterialien sind funktionelle Gruppen immobilisiert.

Vorzugsweise handelt es sich bei den Trägermaterialien um Partikel, wobei es bei dem bevorzugten schichtförmigen Aufbau der Anordnung aus ersten und zweiten Membranelementen von Vorteil ist, wenn die Partikel in ein fluiddurchlässiges Vlies, eine Flachmembran oder ähnliches eingebracht sind. Unter den Begriff Partikel sind im Rahmen der vorliegenden Erfindung auch z.B. Zellen zu verstehen, die in spezifischer Weise mit an sie herangeführten Zielsubstanzen wechselwirken.

Ebenfalls bevorzugte Trägermaterialien sind semipermeable poröse Flachmembranen, die auch ein- oder mehrlagig sein können. Die Trägermaterialien können auch in Form von textilen Flächengebilden vorliegen oder ebenfalls aus Hohlfasermembranmatten bestehen, bei denen die Hohlfasermembranen jedoch an ihren beiden Enden z.B. durch die Einbettung verschlossen sind. Insbesondere diejenigen der genannten Trägermaterialien, die selbst flächig bzw. schichtförmig ausgebildet sind, lassen sich hervorragend in Kombination mit dem bevorzugten schichtförmigen Aufbau der Anordnung aus ersten und zweiten Membranelementen einsetzen. Hierbei ist es zweckmäßig, diese flächigen Trägermaterialien zusammen mit den ersten und zweiten Membranelementen in die Vergussmassen einzubetten. Insbesondere für den Fall, dass eine Flachmembran als Trägermaterial eingesetzt wird, kann dadurch zwischen ersten und zweiten Membranelementen eine weitere im dead-end-Modus betriebene Behandlungsstufe erhalten werden, so dass sich an die cross-flow Filtration in den ersten Membranelementen eine zweifache dead-end Filtration anschließt. Dabei kann die als Trägermaterial eingesetzte Flachmembran neben ihrer auf Wechselwirkung mit Zielsubstanzen ausgerichteten Funktion auch eine fraktionierende Funktion ausüben.

Natürlich ist es auch möglich, dass auf analoge Weise zwischen die ersten und zweiten Membranelemente Flachmembranen als dead-end Filter eingebracht werden, die nicht als Trägermaterial für eine Matrix sondern allein als dead-end Filtrationsstufe dienen und die Aufgabe der Fraktionierung nach Teilchengröße haben.

In dem bevorzugten Fall, dass nur das Trägermaterial Matrix ist, strömt das zu behandelnde Fluid aus dem Verteilerraum durch die Hohlräume der ersten Membranelemente, wobei ein Teilstrom als Permeat durch die Wände der ersten Membranelemente strömt, und nach Austritt aus diesen Wänden auf die gesamte Matrix, d.h. das gesamte Trägermaterial verteilt wird, die es nachfolgend durchströmt. Dabei wird das Fluid stoffspezifisch behandelt. Anschließend tritt das behandelte Fluid durch die Wände der angrenzenden zweiten Membranelemente in die Hohlräume dieser zweiten Membranelemente ein und strömt aus diesen Hohlräumen in den angrenzenden Sammelraum. Dort wird der stoffspezifisch behandelte Permeatstrom mit dem aus den Hohlräumen der ersten Membranelemente ausströmenden Retentatstrom zum stoffspezifisch behandelten Fluid vereinigt, welches dann aus dem Gehäuse über die Auslasseinrichtung abgeleitet wird.

Hierbei kann sich die Funktion der ersten Membranelemente auf die eines reinen Verteilers beschränken, mittels dessen das zu behandelnde Fluid in vorteilhafter Weise gleichmäßig über das gesamte Trägermaterial verteilt wird, und die Funktion der zweiten Membranelemente auf diejenige eines Sammlers, mittels dessen das behandelte Permeat gleichmäßig aus dem gesamten Trägermaterial abgezogen wird. Auf diese Weise wird eine gleichmäßige Nutzung der gesamten auf und/oder im Trägermaterial immobilisierten funktionellen Gruppen erreicht.

Es kann jedoch von den ersten Membranelementen auch gleichzeitig eine stoffspezifische Behandlung in Form einer Fraktionierung erfolgen, so dass von den ersten Membranelementen z.B. nur Moleküle oder Teilchen unterhalb einer bestimmten Größe durchgelassen und der stoffspezifischen Behandlung im Trägermaterial zugeführt werden, wohingegen größere Partikel zurückgehalten und von der stoffspezifischen Behandlung im Trägermaterial ausgeschlossen werden. Ebenso können die zweiten Membranelemente neben ihrer Sammelfunktion auch dazu dienen, über einen Größenausschluss z.B. im Außenraum eingeschlossene partikuläre Bestandteile, die als Matrix dienen, zurückzuhalten.

In einer weiteren vorteilhaften Ausführungsform der erfindungsgemäßen Vorrichtung sind sowohl die ersten und die zweiten Membranelemente als auch die Trägermaterialien Matrix für die funktionellen Gruppen. In diesem Fall kommt also den ersten und zweiten Membranelementen ebenfalls neben ihrer Verteiler- bzw. Sammelfunktion auch eine Funktion als stoffspezifisch behandelnde Elemente zu. Hierbei können die in den Membranelementen und die im Trägermaterial immobilisierten funktionellen Gruppen gleich sein oder auch verschieden. In einer vorteilhaften Ausgestaltung des erfindungsgemäßen Verfahrens wird das zu behandelnde Fluid bei der Durchströmung der Matrix, d.h. bei der Durchströmung der ersten und zweiten Membranelemente sowie des Trägermaterials, nacheinander mindestens zwei verschiedenen stoffspezifischen Behandlungen unterworfen. Natürlich ist es auch möglich, dass außer dem Trägermaterial nur die ersten oder nur die zweiten Membranelemente Matrix für stoffspezifisch wirkende Gruppen sind.

Zur Erhöhung des Wirkungsgrades der stoffspezifischen Behandlung ist es von Vorteil, wenn beim erfindungsgemäßen Verfahren die Schritte b) bis d) mehrfach durchlaufen werden, wobei es besonders vorteilhaft ist, wenn die Schritte b) bis d) innerhalb desselben Membranmoduls mehrfach durchlaufen werden. Bei einer bevorzugten Ausführungsform des erfindungsgemäßen Membranmoduls sind daher im Gehäuse zwischen Einlasseinrichtung und Auslasseinrichtung, d.h. in Richtung der Durchströmung des Gehäuses mehrere, beispielsweise bis zu 100 Membranelementeanordnungen als Modulstufen hintereinander angeordnet. Durch die Hintereinanderschaltung einer Mehrzahl von Modulstufen kann gleichzeitig die einzelne Modulstufe in ihrer Abmessung in Richtung der Durchströmung des Gehäuses kurz gehalten und dadurch eine Konzentrationsänderung bezüglich möglicher kritischer Komponenten in dem zu behandelnden Fluid gering gehalten werden. Dies ist insbesondere bei solchen Verfahren zur stoffspezifischen Behandlung von Suspensionen von Bedeutung, bei denen zumindest ein Teil der suspendierten Teilchen durch die semipermeable Membranwand z.B. der ersten Membranelemente zurückgehalten werden soll und gleichzeitig zu hohe Aufkonzentrationen an suspendierten Teilchen vermieden werden sollen, wie z.B. bei der stoffspezifischen Behandlung von Blut. Durch vergleichsweise kurze Anordnungen von Membranelementen wird entlang der ersten Membranelemente dem zu behandelnden Fluid nur ein geringer Teilstrom entzogen, so dass die Konzentrationsänderungen bis zur folgenden Zusammenführung von Retentatstrom und Permeatstrom gering bleiben.

Bevorzugt sind beim erfindungsgemäßen Membranmodul bis zu 10 Modulstufen im Gehäuse hintereinander angeordnet. Besonders bewährt hat sich eine Modulstufenzahl von bis zu 3. Dabei ist es vorteilhaft, wenn die einzelnen Modulstufen einen Abstand zueinander aufweisen, um eine gute Durchmischung von Retentat- und Permeatstrom in den jeweiligen Sammelräumen zu ermöglichen. Eine gleichmäßige Durchmischung ist von Vorteil, um z.B. unerwünschte Konzentrationsschwankungen zu vermeiden.

Natürlich ist in Anpassung an die Erfordernisse des Behandlungsverfahrens auch die Hintereinanderschaltung mehrerer erfindungsgemäßer Membranmodule möglich, die vorzugsweise mehrere Modulstufen enthalten. Auch ist die Integration der erfindungsgemäßen Module mit anderen Modulen wie z.B. solchen zur Dialyse, zur Hemodiafiltration, zur Hemofiltration, zum Wärmeaustausch oder zu anderen Prozessschritten je nach Anwendung sinnvoll.

In einer weiteren bevorzugten Ausführung des erfindungsgemäßen Verfahrens wird zumindest ein Teil des behandelten Fluids rezirkuliert, d.h. das den Membranmodul verlassende behandelte Fluid wird erneut als Speisestrom, also als zu behandelndes Fluid, dem Modul zugeführt. Das Fluid durchläuft so das stoffspezifische Behandlungsverfahren mehrfach, wodurch die Verweilzeit des Fluids erhöht und der gewünschte Behandlungsgrad eingestellt werden kann.

In dem Fall, bei dem die stoffspezifische Behandlung über funktionelle Gruppen erfolgt, die auf und/oder in einer Matrix immobilisiert sind, transportiert das zu behandelnde Fluid die Zielsubstanz oder die Zielsubstanzen vorzugsweise konvektiv durch die Matrix. Dies setzt voraus, dass die ersten Membranelemente, die zweiten Membranelemente und/oder das Trägermaterial z.B. in Form einer semipermeablen Flachmembran mit poröser Struktur eine Porengröße aufweisen, die einen konvektiven Transport des zu behandelnden Fluid einschließlich der Zielsubstanz bzw. der Zielsubstanzen zulässt. Im Anwendungsfall muss die Porengröße auch auf die Größe der Zielsubstanz bzw. Zielsubstanzen abgestimmt werden, die in Form gelöster Moleküle oder Makromoleküle, aber auch in Form kleiner Teilchen mit einer Teilchengröße im Sub-Mikrometerbereich vorliegen kann, so dass kein Rückhalt der Zielsubstanz bzw. -substanzen aufgrund ihrer Größe erfolgt.

Im Hinblick auf den Einsatz des erfindungsgemäßen Membranmoduls, insbesondere dann, wenn Membranelemente oder eine Flachmembran als Matrix dienen, ist es andererseits wichtig, Membranen mit möglichst kleinen Porengrößen und möglichst großem Porenvolumen bzw. möglichst großer Porosität zu verwenden, um so für die stoffispezifische Behandlung eine möglichst große innere Oberfläche der Membranen zur Verfügung zu stellen. Bevorzugt weisen die erfindungsgemäß eingesetzten Membranen eine mittlere Porosität zwischen 50 Vol.-% und 90 Vol.-% auf. Als mittlere Porosität wird das Verhältnis des Porenvolumens der Membran zum Membranwandvolumen verstanden, wobei sich das Membranwandvolumen aus dem Porenvolumen und dem Volumen des die Membranstruktur aufbauenden Materials zusammensetzt. Letztlich muss bei Ausführungsformen der erfindungsgemäßen Vorrichtung, die Trägermaterialien in Form von Partikeln enthalten, die Porengröße der Membranelemente so auf die Größe der Partikel abgestimmt sein, dass die Partikel durch die Membranelemente zurückgehalten werden. Die Membranen sind also in diesen Fällen vorteilhafterweise im wesentlichen durchlässig für die Zielsubstanz bzw. Zielsubstanzen und im wesentlichen undurchlässig für die Partikel des Trägermaterials. Dabei können die ersten und zweiten Membranelemente auch unterschiedliche Porengrößen aufweisen, insbesondere dann, wenn die erfindungsgemäße Vorrichtung immer in der gleichen Richtung durchströmt wird.

Die Anforderungen an den Aufbau der Membranen, d.h. an ihre Struktur und Porengrößenverteilung über der Membrandicke resultieren aus dem jeweiligen Anwendungsfall der stoffspezifischen Behandlung. Die Membranstruktur kann über die Dicke isotrop sein, d.h. innerhalb der Membranstruktur sind die Porendurchmesser im wesentlichen konstant, sie kann aber auch anisotrop, symmetrisch oder auch asymmetrisch sein. Hierbei werden unter im wesentlichen isotropen Porenstrukturen solche Strukturen verstanden, bei denen sich die Porengröße in den Erstreckungsrichtungen der Membranen um maximal etwa den Faktor 10 ändert. Derartige im wesentlichen isotrope Strukturen zeichnen sich dadurch aus, dass sie eine große innere Oberfläche aufweisen, an der eine große Anzahl von stoffspezifisch wirkenden Gruppen immobilisiert werden kann. Des Weiteren führt eine solche Struktur in der Anwendung zu einer gleichmäßigen Nutzung dieser Gruppen. Daher sind für Ausführungsformen des erfindungsgemäßen Membranmoduls, bei denen die eingesetzten Membranen Matrix für die funktionellen Gruppen sind, Membranen mit isotroper Struktur bevorzugt.

Für den Fall, dass die ersten Membranelemente eine fraktionierende Funktion aufweisen und die zweiten Membranelemente Matrix für funktionelle Gruppen sind, werden bevorzugt für die ersten Membranelemente Membranen mit asymmetrischer Struktur eingesetzt, die eine hohe hydraulische Permeabilität bei hoher Trennschärfe für die abzutrennenden partikulären Bestandteile des Fluids haben, und für die zweiten Membranelemente Membranen im wesentlichen isotroper Porenstruktur.

In dem erfindungsgemäßen Membranmodul bzw. bei Durchführung des erfindungsgemäßen Verfahrens werden bevorzugt Membranen mit einem mittleren Porendurchmesser zwischen 0,01 und 10 µm eingesetzt. Für den Fall, dass der jeweiligen Membran eine fraktionierende Funktion zukommt, richtet sich die Porengröße der Trennschicht nach der Größe der abzutrennenden partikulären Bestandteile. In der Regel weisen diese Membranen in der Trennschicht einen mittleren Porendurchmesser zwischen 0,01 und 1 µm und in der darunterliegenden Stützstruktur einen mittleren Porendurchmesser zwischen 0,2 und 10 µm auf. In den Fällen, in denen die jeweilige Membran Matrix für funktionelle Gruppen ist, besitzt sie vorzugsweise einen mittleren Porendurchmesser zwischen 0,2 und 10 µm.

Zur Bestimmung des mittleren Porendurchmessers werden je nach Größe des Porendurchmessers und je nach Membranstruktur unterschiedliche Verfahren angewandt. Für im wesentlichen isotrope Porenstrukturen werden Porendurchmesser indirekt durch ein Filtrationsexperiment bestimmt, indem eine wässrige Dextranlösung mit einer vorgegebenen Größenverteilung von Dextranmolekülen durch die Membran filtriert wird. Aus dem dabei gemessenen relativen Rückhalt als Funktion der nominalen Moleküldurchmesser wird die Porendurchmesserverteilung und daraus der mittlere Porendurchmesser berechnet. Dieses Verfahren wird beispielsweise von K. Sakai, J. Membrane Science 96 (1994), 91-130, oder von Shin-ichi Nakao, J. Membrane Science 96 (1994) 131-165, für Dialyse- bzw. Filtrationsmembranen beschrieben.

Für anisotrope Membranen, die z.B. eine Schicht mit dichterer Porenstruktur aufweisen, werden zur Bestimmung der mittleren Porendurchmesser innerhalb der dichteren Schicht ebenfalls die zitierten Bestimmungsverfahren basierend auf Filtrationsexperimenten herangezogen. Zur Bestimmung der mittleren Porendurchmesser der grobporigeren Bereiche der anisotropen Membranen wird ein bildanalytisches Verfahren nach L. Zeman u.a., J. Membrane Science 71 (1992), 221-231 eingesetzt. Dieses eignet sich für einen Porengrößenbereich zwischen 0,1 µm und 10 µm, naturgemäß sowohl für isotrope als auch für anisotrope Porenstrukturen.

In den Fällen, in denen die ersten Membranelemente und/oder die zweiten Membranelemente und/oder die gegebenenfalls als Trägermedium eingesetzten Membranen Matrix für funktionelle Gruppen sind, werden in der erfindungsgemäßen Vorrichtung bzw. zur Durchführung des erfindungsgemäßen Verfahrens vorzugsweise als Matrix poröse Membranen mit großer innerer Oberfläche eingesetzt. Bewährt haben sich poröse Membranen mit einer BET-Oberfläche zwischen 2 und 300 m² je cm³ Membranvolumen, bestens bewährt haben sich solche Membranen mit einer BET-Oberfläche zwischen 4 und 30 m² je cm³ Membranvolumen. Das auf Stickstoffadsorptionsmessung basierende BET-Verfahren zur Bestimmung der Oberfläche poröser Membranstrukturen ist von K. Kaneko, J. Membrane Science 96 (1994), 59-89 beschrieben.

Abhängig von der gewünschten Anwendung der erfindungsgemäßen Vorrichtung bzw. des erfindungsgemäßen Verfahrens können die für die ersten Membranelemente, die für die zweiten Membranelemente sowie die gegebenenfalls als Trägermaterial eingesetzten Membranen gleich sein oder auch unterschiedlich. Unterschiede können sich beispielsweise auf ihre Porenstruktur oder ihre Porendurchmesser beziehen, sie können sich jedoch auch auf in den Membranen enthaltene funktionelle Gruppen beziehen, die zur Wechselwirkung mit in dem zu behandelnden Fluid enthaltenen Zielsubstanzen bestimmt sind.

Für den Fall, dass als Trägermaterial Partikel eingesetzt werden, sind diese zweckmäßigerweise - wie ausgeführt - z.B. in ein fluiddurchlässiges Vlies, eine Flachmembran oder ähnliches eingebracht, etwa um die Position der Partikel zwischen den ersten und zweiten Membranelementen zu stabilisieren oder um im bevorzugten Fall, dass die Anordnung aus ersten und zweiten Membranelementen einen schichtförmigen Aufbau aufweist, eine gleichmäßige Verteilung der Partikel zwischen den Schichten oder auch einen gleichmäßigen Abstand zwischen den Schichten zu erreichen. Das fluiddurchlässige Vlies oder z.B. die Flachmembran kann dabei eine relativ grobe Struktur aufweisen; wesentlich ist allein, dass die Partikel problemlos in das Vlies oder die Flachmembran eingebracht werden können und dort im späteren Einsatz stabil positioniert sind.

Derartige Partikel können z.B. solche sein, wie sie gemäß dem Stand der Technik in Säulen zur Affinitätschromatographie eingesetzt werden. Hierbei kann die Struktur der Partikel dicht sein, so dass im zu behandelnden Fluid enthaltene Zielsubstanzen z.B. nur durch einen Lösevorgang oder durch Diffusion zu im Inneren der Partikel immobilisierten stoffspezifisch wirkenden Gruppen gelangen können. Bevorzugt weisen die Partikel jedoch eine poröse Struktur auf, und ein konvektiver Transport der Zielsubstanzen zu den immobilisierten stoffspezifisch wirkenden Gruppen auch an der inneren, durch die Poren definierten Oberfläche ist möglich. Der Porendurchmesser dieser Partikel liegt vorzugsweise im Bereich zwischen 0,01 und 10 µm und besonders bevorzugt zwischen 0,1 und 3 µm.

Unter Partikeln werden aber auch Makromoleküle wie Proteine (z.B. Albumin), an die beispielsweise kleinere Moleküle (z.B. Bilirubin) gebunden sein können, oder, wie ausgeführt, auch Zellen verstanden. Die Gestalt der Partikel kann kugelförmig sein oder jede andere Form annehmen, wie z.B. ellipsoidisch oder stäbchenförmig.

Beispielsweise zur Einbringung der Partikel oder aber auch zur Entlüftung des Außenraums um die Membranelemente ist es zweckmäßig, den erfindungsgemäßen Membranmodul mit einer oder mehreren verschließbaren Aus- bzw. Einlassöffnungen in der Gehäusewand zu versehen.

Hinsichtlich des Materials, aus dem die Membranelemente und gegebenenfalls als Trägermaterial eingesetzten Flachmembranen gemäß der Erfindung aufgebaut sind, sind keinerlei Einschränkungen gegeben. So können Membranen aus anorganischen Materialien wie Glas, Keramik, SiO₂, Kohlenstoff oder Metall, aus organischen Polymeren oder Mischungen daraus eingesetzt werden. Die Polymeren können hydrophilen und/oder hydrophoben Charakter aufweisen, sie können ausgewählt sein aus der Gruppe der cellulosischen Polymeren, wie z.B. Cellulose oder regenerierte Cellulose, modifizierte Cellulose, wie z.B. Celluloseester, Celluloseäther, aminmodifizierte Cellulosen, sowie Mischungen von cellulosischen Polymeren, aus der Gruppe der synthetischen Polymeren wie z.B. Polyacrylnitril und entsprechende Copolymere, Polyurethan enthaltende Polymere, Polyarylsulfone und Polyarylethersulfone, wie z.B. Polysulfon oder Polyethersulfon, Polyvinylidenfluorid, Polytetrafluorethylen, wasserunlösliche Polyvinylalkohole, aliphatische und aromatische Polyamide, Polyimide, Polyetherimide, Polyester, Polycarbonate, Polyolefine, wie Polyethylen, Polypropylen, Polyvinylchlorid, Polyphenylenoxid, Polybenzimidazole und Polybenzimidazolone, sowie daraus gewonnenen Modifikationen, Blends, Mischungen oder Copolymeren dieser Polymeren. Diesen Polymeren bzw. Polymergemischen können weitere Polymere wie z.B. Polyethylenoxid, Polyhydroxyether, Polyethylenglykol, Polyvinylpyrrolidon, Polyvinylalkohol oder Polycaprolacton, oder anorganische Stoffe wie z.B. SiO₂ als Zusatzstoffe beigemischt werden. Im Einzelfall kann die Membran auch z.B. einer Oberflächenmodifikation unterzogen worden sein, um bestimmte Eigenschaften der Membranoberfläche z.B. in Form bestimmter funktioneller Gruppen einzustellen. Im Falle des Einsatzes von polyolefinischen Polymeren kann es erforderlich sein, zumindest die innere Oberfläche der Membran z.B. mit einem funktionalisierbaren Polymer zu beschichten.

Besonders gute Erfahrungen wurden mit Membranen aus cellulosischen Polymeren, Polyamiden, Polypropylen, Polyethersulfonen oder aus lösemittelstabilen und pH-Wert-stabilen Polymeren gemacht, insbesondere mit Membranen aus Polytetrafluorethylen oder Polyvinylidenfluorid, sowie daraus gewonnenen Modifikationen, Blends, Mischungen oder Copolymeren. Derartige Membranen werden beispielsweise in der DE-A-39 23 128 beschrieben.

Der erfindungsgemäße Membranmodul bzw. das erfindungsgemäße Verfahren kann mit Erfolg zu verschiedensten stoffspezifischen Behandlungen von Fluiden verwendet werden. Bei Membranmodulen, die eine Matrix enthalten, können je nach dem jeweiligen Behandlungsverfahren auch mehrere verschiedene funktionelle Gruppen auf und/oder in der Matrix immobilisiert sein, die spezifisch mit verschiedenen Zielsubstanzen wechselwirken. Es können auch für die ersten und zweiten Membranelemente und die gegebenenfalls als Trägermaterial eingesetzte Flachmembran unterschiedliche Membranen mit unterschiedlichen stoffspezifisch wirkenden Gruppen zusammen eingesetzt werden, wenn es die Anwendung erfordert. Auf diese Weise können in der Matrix unterschiedliche stoffspezifische Behandlungen erfolgen. In anderen Fällen lässt sich auch eine solche Behandlung des Fluids über Wechselwirkung mit den funktionellen Gruppen kombinieren mit einer Vorfiltration des Fluids, die dann in den ersten Membranelementen erfolgt, um so z.B. bestimmte Bestandteile aus dem zu behandelnden Fluid zurückzuhalten, die nicht mit den funktionellen Gruppen in Kontakt kommen sollen. Bei Einsatz von Membranmodulen mit mehreren Modulstufen können in den einzelnen Modulstufen gleiche, jedoch auch unterschiedliche funktionelle Gruppen immobilisiert sein.

Zur Immobilisierung der funktionellen Gruppen auf und/oder in der Matrix können die in der Literatur beschriebenen Verfahren eingesetzt werden. Auch hinsichtlich der in Bezug auf die jeweilige stoffspezifische Fluidbehandlung verwendbaren funktionellen Gruppen kann auf die in der Literatur beschriebenen zurückgegriffen werden. Verschiedene Möglichkeiten der Immobilisierung der funktionellen Gruppen kommen in Betracht, sowohl in Bezug auf den Ort als auch in Bezug auf die Art und Weise ihrer Immobilisierung.

So können diese funktionellen Gruppen adsorptiv oder über kovalente Bindungen an die Matrix gekoppelt sein. Diese Kopplung an die Matrix kann sowohl vor Einbau in das Gehäuse als auch nach Einsetzen der Matrix in das Gehäuse des erfindungsgemäßen Membranmoduls erfolgen. Hierbei können in Abstimmung auf den jeweiligen Anwendungsfall die funktionellen Gruppen beispielsweise im wesentlichen homogen an die gesamte Oberfläche der Matrix, d.h. sowohl an die äußeren als auch an die inneren, z.B. durch die Poren gebildeten Oberflächen gekoppelt, d.h. auf und in der Matrix immobilisiert sein. Es kann aber auch erforderlich sein, dass die funktionellen Gruppen nur an einem Teil dieser Oberflächen immobilisiert sind, etwa wenn einzelne Bestandteile des zu behandelnden Fluids nicht mit den funktionellen Gruppen in Kontakt kommen sollen.

Auch ein direkter Einbau von funktionellen Gruppen in das Matrixmaterial ist möglich, im Falle von Matrices aus polymeren Materialien etwa durch Modifikation des Polymermaterials mit beispielsweise ionischen, hydrophilen oder hydrophoben Gruppen oder durch Einsatz von Polymerblends, bei denen mindestens eine Polymerkomponente funktionelle, d.h. stoffspezifisch wirkende Gruppen aufweist.

Eine weitere Möglichkeit besteht darin, derartige funktionelle Gruppen oder auch solche Gruppen aufweisende Trägersubstanzen oder Partikel in das Porensystem einer Membran beim Herstellungsverfahren der Membran einzulagern oder nachträglich in die fertige Membran z.B. einzuschwemmen. In letzterem Fall weist die Membran zweckmäßigerweise eine asymmetrische Struktur sowie gegebenenfalls eine Haut auf, wobei die Öffnungen der Haut bzw. die Poren der feinporigeren Seite der Membran so bemessen sind, dass die funktionellen Gruppen bzw. die genannten Trägersubstanzen oder Partikel nicht hindurchtreten können.

Vorzugsweise ist die Porengröße der verwendeten Membran so zu wählen, dass auch trotz der in den Poren immobilisierten funktionellen Gruppen die Zielsubstanzen konvektiv von zumindest einem Teil des zu behandelnden Fluids durch die Membranwand transportiert werden können.

Gemäß einer besonders bevorzugten Ausgestaltung der Erfindung handelt es sich bei den funktionellen Gruppen um Liganden zur affinen Trennung von Ligaten aus zu behandelnden Flüssigkeiten oder um Katalysatoren, wobei unter Katalysatoren auch Biokatalysatoren wie z.B. Enzyme zu verstehen sind. Bevorzugte erfindungsgemäße Verfahren sind Verfahren zur Reinigung/Trennung von Ligaten aus einer ligathaltigen Flüssigkeit, wobei eine Matrix ausgewählt wird, auf und/oder in der Liganden für besagte Ligaten immobilisiert sind, bzw. erfindungsgemäße Membranmodule verwendet werden, die derartige Matrices enthalten. Des Weiteren sind bevorzugte Verfahren solche zur katalytischen Behandlung von Flüssigkeiten,
wobei eine Matrix ausgewählt wird, auf und/oder in der Katalysatoren immobilisiert sind, bzw. erfindungsgemäße Membranmodule verwendet werden, die derartige Matrices enthalten. Zu den katalytischen Verfahren zählen auch biokatalytische Verfahren wie z.B. enzymatische Verfahren.

Liganden können in dem hier gebrauchten Sinne je nach Anwendung nicht-spezifisch, gruppenspezifisch oder spezifisch wirken. Hinsichtlich einsetzbarer Liganden sowie hinsichtlich der Möglichkeiten ihrer Immobilisierung sei auf die Ausführungen in der europäischen Patentanmeldung EP-A-0 787 523 verwiesen, auf deren diesbezügliche Offenbarung sich hier ausdrücklich bezogen wird.

Ohne an dieser Stelle die Möglichkeiten erschöpfend aufzuzählen, können die Liganden z.B. durch Modifikation der Oberflächen der Matrix erzeugt werden, sie können direkt oder über Abstandsmoleküle (Spacer), sie können aber auch über Tentakelsysteme oder Ketten an die Oberfläche gebunden werden, wobei an jede Kette bzw. an jedes Tentakelsystem mehrere Liganden gebunden sein können.

Um die Kapazität insbesondere von lonenaustauschermatrices zu erhöhen, sind verschiedene an sich bekannte Methoden anwendbar, wobei die Anzahl der stoffspezifisch wirkenden Gruppen, d.h. der Liganden, auf der Oberfläche der Matrices erhöht wird. Bevorzugt sind die Liganden über Moleküle langkettiger Linearpolymerer an die Membran gekoppelt, wobei die Moleküle der langkettigen Linearpolymeren eine Mehrzahl von Liganden tragen. Die Verwendung langkettiger Linearpolymerer mit Seitenarmen, sogenannter Tentakeln, wobei die Liganden an den Seitenarmen sitzen, beschreibt z.B. W. Müller, J. Chromatogr., Bd. 510 (1990), S. 133. Die Herstellung solcher Tentakel ist beispielsweise bei Tsuneda u.a. (Biotechnol. Prog., Bd. 10 (1994), S. 76-81, und J. Chromatogr. Bd. A 689 (1995), S. 211-218) beschrieben und kann über eine strahleninduzierte Pfropfpolymerisation von einem eine Epoxidgruppe enthaltenden Monomer, wie z.B. Glycidylmethacrylat, mit anschließender chemischer Umsetzung in SO₃H-Gruppen oder Diethylamino-Gruppen erfolgen. Ein anderes Verfahren zur Pfropfung von stickstoffhaltigen polymeren Flachmembranen, das zur Erhöhung der lonenaustauscherkapazität der erfindungsgemäßen Membranelemente eingesetzt werden kann, wird in der EP-A-0 490 940 beschrieben.

Membranen, die mit polymerisierbaren Doppelbindungen derivatisierte Polyamide gemäß der DE-OS-195 01 726 enthalten, sind für die erfindungsgemäße Vorrichtung bzw. zur Durchführung des erfindungsgemäßen Verfahrens bestens geeignet.

Der erfindungsgemäße Membranmodul bzw. das erfindungsgemäße Verfahren ist für zahlreiche Anwendungen zur Reinigung/Trennung von Ligaten aus einer ligathaltigen Flüssigkeit einsetzbar, wie sie beispielsweise allgemein aus dem Bereich der Affinitätschromatographie bekannt sind, wobei unter Affinitätschromatographie biospezifische Adsorptionen oder auch die lonenaustauscherchromatographie oder die Metallchelatchromatographie verstanden werden.

Interessante Anwendungen beziehen sich auf die Reinigung von monoklonalen Flüssigkeiten, auf die Entfernung von Proteasen zur Stabilisierung von biologischen Flüssigkeiten, auf die Gewinnung oder therapeutische Entfernung von Blutplasmabestandteilen aus Blutplasma oder Vollblut. So kann zur Behandlung von Vollblut ein Hemodialysator oder ein Hemodiafilter mit einem erfindungsgemäßen Membranmodul in Reihe geschaltet oder direkt mit ihm gekoppelt werden. Im Hemodialysator bzw. Hemodiafilter werden dann harnpflichtige Substanzen auf übliche Weise entfernt. Im sich anschließenden erfindungsgemäßen Membranmodul, in dem das Vollblut im cross-flow Modus durch die ersten Membranelemente geleitet wird, können dann mittels in diesem Modul immobilisierter, entsprechend ausgewählter funktioneller Gruppen selektiv z.B. Cytokine, albumingebundene Toxine oder allgemein Toxine mit einem Molekulargewicht von größer als 10000 D, d.h. solche Substanzen, deren Entfernung mittels der Hemodialyse oder der Hemodiafiltration nur in unzureichendem Maße möglich ist, aus dem Blut entfernt werden.

Andere Anwendungen sind die Entfernung von Pyrogenen aus biologischen oder pharmazeutischen Flüssigkeiten, die Trennung von Enantiomeren, die Isolierung von Enzymen oder auch die Zellselektion mittels spezifischer, auf ein bestimmtes Oberflächenprotein der Zellen reagierender Liganden, um nur einige Beispiele zu nennen. Auch für Anwendungen im Bereich der Gentechnik sind die erfindungsgemäßen Membranmodule bestens geeignet, wenn bei solchen Anwendungen z.B. ein konvektiver Transport von Genen zu beispielsweise auf und/oder in der Membran immobilisierten Viren oder Zellen erreicht werden soll.

In einer Ausführungsform lässt sich der erfindungsgemäße Membranmodul als Heparinadsorber für Vollblut einsetzen. In diesem Fall dienen die ersten Membranelemente, die vom Vollblut durchströmt werden, vorzugsweise allein der Plasmaseparation, und die Heparinadsorption als stoffspezifische Behandlung erfolgt über im Außsenraum immobilisierte Trägermaterialien und oder in den zweiten Membranelementen. Vorzugsweise erfolgt die Heparinadsorption jedoch allein in den zweiten Membranelementen, die mit Tentakeln versehen sind, die DEA-(Diethylamino-) Gruppen als Liganden tragen. In dieser Ausgestaltung sind die ersten und zweiten Membranelemente bevorzugt so zueinander angeordnet, dass der Außenraum klein gehalten ist. Die Trennung von Plasmaseparation und Heparinadsorption erlaubt die separate Optimierung der für die jeweiligen Membranelemente eingesetzten Membranen. Dies ist auch deshalb von Vorteil, da dann für die Plasmaseparation relativ preiswerte Membranen eingesetzt werden können und die Verwendung der für die Heparinadsorption modifizierten Membranen, die wegen der aufwendigen Modifizierungsverfahren relativ teuer sind, auf die zweiten Membranelemente beschränkt bleibt.

Wie ausgeführt können im Außenraum um die ersten und zweiten Membranelemente Partikel als Matrix eingebracht sein, wobei unter Partikel im Rahmen der vorliegenden Erfindung auch z.B. Zellen zu verstehen sind, die in spezifischer Weise mit an sie herangeführten Zielsubstanzen wechselwirken. In einer weiteren Ausführungsform des erfindungsgemäßen Membranmoduls lässt sich dieser damit als künstliches Organ, beispielsweise als künstliche Leber einsetzen. In diesem Fall werden über eine Öffnung im Gehäuse z.B. tierische Hepatozyten in den Außenraum um die ersten und zweiten Membranelemente eingebracht und dort immobilisiert. Es ist dabei günstig, wenn die Membranelementeanordnung einen schichtförmigen Aufbau aufweist, wobei die einzelnen Schichten jeweils nur erste bzw. nur zweite Membranelemente enthalten. Darüber hinaus ist es zweckmäßig, zwischen die Schichten jeweils ein Vlies als Abstandshalter einzubringen, wobei das Vlies so gestaltet sein kann, dass es gleichzeitig Ankerpunkt für die Hepatozyten ist. In der Anwendung reinigen die Hepatozyten Blut oder Blutplasma, welches im cross-flow-Modus durch die ersten Membranelemente strömt, von Stoffwechselprodukten und Giften. Gleichzeitig werden die Hepatozyten über die ersten Membranelemente mit Sauerstoff und Nährstoffen versorgt. Die zweiten Membranelemente dienen dazu, die Hepatozyten zurückzuhalten, während der von den Hepatozyten gereinigte Plasmastrom diese Membranelemente durchströmt. Dieser gereinigte Plasmastrom wird nach Ausströmen aus den Hohlräumen der zweiten Membranelemente mit dem aus den ersten Membranelementen ausströmenden Blut bzw. Blutplasma zusammengeführt.

Für Anwendungen im Bereich der enzymatischen oder allgemein katalytischen Behandlung von Flüssigkeiten können Hohlfasermembranen ausgewählt werden, auf und/oder in denen nach an sich bekannten Methoden Enzyme oder Katalysatoren immobilisiert sind. Hinsichtlich der mit dem erfindungsgemäßen Membranmodul bzw. dem erfindungsgemäßen Verfahren durchführbaren katalytischen Behandlungen sei wiederum auf die Ausführungen in der europäischen Patentanmeldung EP-A-0 787 523, verwiesen, auf deren diesbezügliche Offenbarung sich hier ausdrücklich bezogen wird.

Die Erfindung wird nachfolgend anhand der Figuren näher erläutert. Es zeigen in vereinfachter schematischer Darstellung:
- Fig. 1:: Membranmodul mit ersten und zweiten Membranelementen in Form von Hohlfäsermembranen
- Fig. 2:: Membranmodul mit ersten und zweiten Membranelementen in Form von Hohlfasermembranen, wobei im Außenraum um die Membranelemente Flachmembranen angeordnet sind
- Fig. 3:: Membranmodul mit ersten und zweiten Membranelementen in Form von Hohlfasermembranen, wobei im Außenraum um die Membranelemente Partikel als Trägermaterial angeordnet sind
- Fig. 4:: Membranmodul mit ersten und zweiten Membranelementen in Form von Hohlfasermembranen, bei dem die ersten Membranelemente im Zentrum des Moduls zusammengefasst und die zweiten Membranelemente ringförmig um die ersten Membranelemente angeordnet sind
- Fig. 5:: Membranmodul, bei dem die ersten Membranelemente aus Hohlfasemnembranen und die zweiten Membranelemente aus U-förmig gefalteten Flachmembranen bestehen.
- Fig. 6:: Membranmodul mit zwei Modulstufen, wobei die ersten Membranelemente aus Hohlfasermembranen und die zweiten Membranelemente aus U-förmig gefalteten Flachmembranen bestehen.

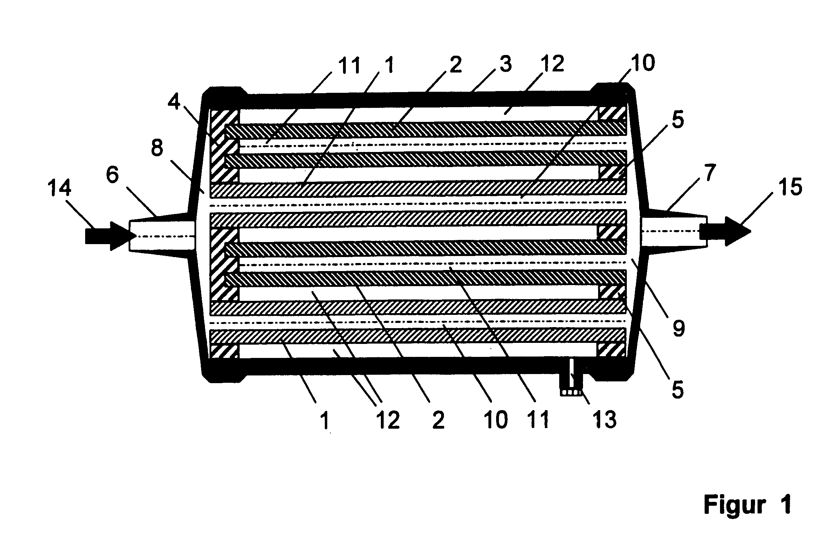

Figur 1 zeigt einen Längsschnitt durch einen erfindungsgemäßen Membranmodul mit ersten Membranelementen 1 und zweiten Membranelementen 2 in Form von Hohlfasermembranen, die in einem Gehäuse 3 in Vergussmassen 4,5 eingebettet sind. Die Hohlfasermembranen sind im dargestellten Fall in ersten Schichten, die nur erste Membranelemente 1 enthalten, und zweiten Schichten, die nur zweite Membranelemente 2 enthalten, angeordnet, wobei sich die Schichten in dieser Darstellung längs der Hohlfasermembranen sowie senkrecht zur Zeichenebene erstrecken. Die ersten und zweiten Schichten sind gemäß Figur 1 in alternierender Reihenfolge übereinandergeschichtet.

Eine solche in der Längsschnittdarstellung alternierende Reihenfolge von ersten Membranelementen 1 und zweiten Membranelementen 2 lässt sich z.B. auch realisieren, indem eine Hohlfasermatte, die nur erste Membranelemente 1 enthält, und eine Hohlfasermatte, die nur zweite Membranelemente 2 enthält, übereinandergelegt werden und miteinander spiralförmig aufgewickelt werden. In solchen Fällen besitzt das Gehäuse 3 vorzugsweise einen runden Querschnitt.

Das Gehäuse 3 weist eine Einlasseinrichtung 6 zum Einleiten des zu behandelnden Fluids in das Gehäuse 3 auf sowie eine Auslasseinrichtung 7 zum Ableiten des behandelnden Fluids aus dem Gehäuse 3. Die Einlasseinrichtung 6 steht mit einem Verteilerraum 8, die Auslasseinrichtung 7 mit einem Sammelraum 9 in Verbindung. Die ersten Membranelemente 1 sind so in die Vergussmassen 4,5 eingebettet, dass sie mit ihren Enden durch die Vergussmassen 4,5 hindurchtreten und an diesen Enden offen sind, wodurch die Hohlräume 10 der ersten Membranelemente 1 sowohl in den Verteilerraum 8 als auch in den Sammelraum 9 münden. Die zweiten Membranelemente 2 sind so in die Vergussmassen 4,5 eingebettet, dass sie an ihrem dem Verteilerraum 8 zugewandten Ende von der Vergussmasse 4 verschlossen werden. An ihrem dem Sammelraum 9 zugewandten Ende treten sie durch die Vergussmasse 5 hindurch und sind an diesem Ende offen. Auf diese Weise sind die Hohlräume 11 der zweiten Membranelemente 2 nur zum Sammelraum 9 hin geöffnet.

Der zwischen den Membranelementen 1,2 und den Vergussmassen 4,5, ausgebildete Außenraum 12 kann im dargestellten Beispiel über eine verschließbare Öffnung 13 befüllt oder entlüftet werden.

In einer Ausgestaltung des erfindungsgemäßen Verfahrens strömt das zu behandelnde Fluid, dargestellt durch den Pfeil 14, als Speisestrom durch die Einlasseinrichtung 6 in den Verteilerraum 8 des Membranmoduls gemäß Figur 1 ein und durchströmt anschließend die Hohlräume 10 der ersten Membranelemente 1 im cross-flow-Modus. Dabei tritt ein Teil des Speisestroms als Permeat durch die porösen Wände der ersten Membranelemente 1 in den Außenraum 12 ein. Der als Retentat verbleibende Teil des Speisestroms verlässt die Hohlräume 10 der ersten Membranelemente 1 an den in der Vergussmasse 5 eingebetteten Enden der ersten Membranelemente 1 und tritt in den Sammelraum 9 ein.

Das Permeat strömt aus dem Außenraum durch die Wände der zweiten Membranelemente 2 in deren Hohlräume 11 ein und verlässt diese über die in der Vergussmasse 5 eingebetteten, geöffneten Enden, um im Sammelraum 9 mit dem Retentat zusammengeführt zu werden. Der aus Permeat und Retentat vereinigte Fluidstrom verlässt als behandeltes Fluid, dargestellt durch den Pfeil 15, über die Auslasseinrichtung 7 den erfindungsgemäßen Membranmodul. Die stoffspezifische Behandlung des zu behandelnden Fluids erfolgt in den Wänden der ersten Membranelemente 1 und/oder im Außenraum 12 und/oder in den Wänden der zweiten Membranelemente 2.

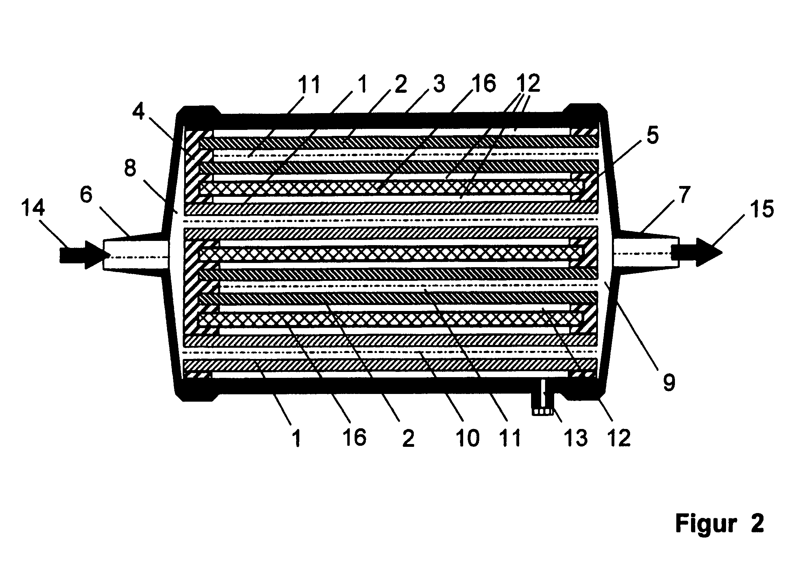

In Figur 2 ist eine Ausführungsform des erfindungsgemäßen Membranmoduls dargestellt, bei der im Außenraum 12 um die Membranelemente 1,2 Flachmembranen 16 angeordnet sind, die gemeinsam mit den Membranelementen 1,2, in die Vergussmassen 4,5 eingebettet sind. Auf diese Weise durchströmt das Permeat auf dem Weg von den ersten Membranelementen 1 zu den zweiten Membranelementen 2 die Flachmembranen 16 im dead-end-Modus. Die Flachmembran ist, wie erläutert, bevorzugt Trägermaterial und Matrix für funktionelle Gruppen. Sie kann jedoch auch allein als dead-end Filtrationsstufe dienen und eine Fraktionierung nach Teilchengröße bewirken.

Im Falle, dass die Anordnung aus ersten und zweiten Membranelementen 1,2 aus spiralförmig aufgewickelten Hohlfasermatten aufgebaut ist, die jeweils nur erste bzw. nur zweite Membranelemente enthalten, lässt sich die Flachmembran 16 im Außenraum leicht dadurch anordnen, dass sie vor dem spiralförmigen Wickeln zwischen die Hohlfasermatten gelegt wird und dann zusammen mit den Hohlfasermatten aufgewickelt wird. Gegebenenfalls kann für Anwendungen, bei denen zwischen den Hohlfasermembranen 1,2 und der Flachmembran 16 ein Abstand erforderlich ist, zwischen die Flachmembran 16 und die jeweilige Hohlfasermatte zusätzlich ein flächiger Abstandshalter gelegt werden, der ebenfalls mit aufgewickelt wird.

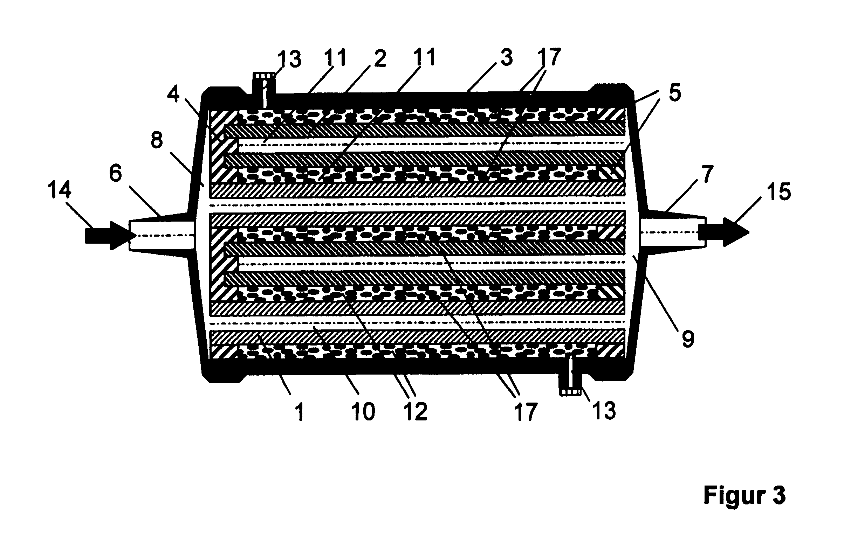

Figur 3 zeigt eine Ausführungsform des erfindungsgemäßen Membranmoduls ähnlich der in Figur 1 dargestellten, bei der jedoch in den Außenraum 12 um die Membranelemente 1,2 Partikel 17 als Trägermaterialien eingebracht sind, die Matrix für funktionelle Gruppen sind. Damit eine stabile Positionierung erreicht wird, können die Partikel auch in ein Vlies eingebettet sein, das gleichzeitig als Abstandshalter zwischen den Schichten aus ersten und zweiten Membranelementen 1,2 dient. Die Partikel 17 lassen sich beispielsweise über die Öffnungen 13 in den Außenraum 12 einfüllen und durch Rotation und/oder Taumelbewegung des Moduls gleichmäßig im Außenraum 12 verteilen.

Unter Partikel werden, wie bereits ausgeführt, z.B. auch Zellen verstanden, die in spezifischer Weise mit an sie herangeführten Zielsubstanzen wechseiwirken. In einer Ausführungsform des erfindungsgemäßen Membranmoduls lässt sich dieser damit als künstliches Organ, beispielsweise als künstliche Leber einsetzen.

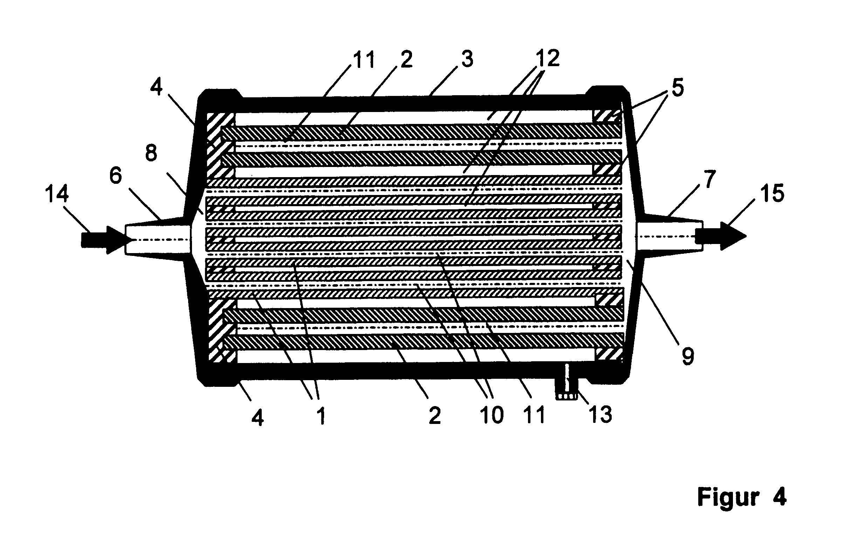

In Figur 4 ist ebenfalls ein erfindungsgemäßer Membranmodul dargestellt, dessen Membranelemente 1,2 aus Hohlfasermembranen bestehen. Der Membranmodul gemäß Figur 4 enthält jedoch eine größere Anzahl an ersten Membranelementen 1 für die cross-flow Filtration im Vergleich zur Anzahl an zweiten Membranelementen 2 für die dead-end Filtration. Im dargestellten Fall ist das Verhältnis N₁/N₂ der Anzahl N₁ der ersten Membranelemente 1 zur Anzahl N₂ der zweiten Membranelemente 2 gleich 4, wobei die ersten Membranelemente 1 im Zentrum des Moduls zusammengefasst sind und die zweiten Membranelemente 2 z.B. in Form einer Hohlfasermatte ringförmig um das Bündel der ersten Membranelemente 1 angeordnet sind.

Generell sind je nach Anwendung des erfindungsgemäßen Membranmoduls unterschiedliche Verhältnisse N₁/N₂ möglich, wobei das Verhältnis vorzugsweise den Bereich 0,5 bis 10 abdeckt. Hinsichtlich der Anordnung sind verschiedene Alternativen denkbar. Bei einem Verhältnis N₁/N₂ von beispielsweise 4 können bei einem stapelförmigen Aufbau der Membranelementeanordnung vier Lagen von Hohlfasermatten mit ersten Membranelementen mit einer Lage einer Hohlfasermatte mit zweiten Membranelementen kombiniert werden. Es können aber auch Hohlfasermatten verarbeitet werden, die bereits erste und zweite Membranelemente im Verhältnis 4:1 enthalten.

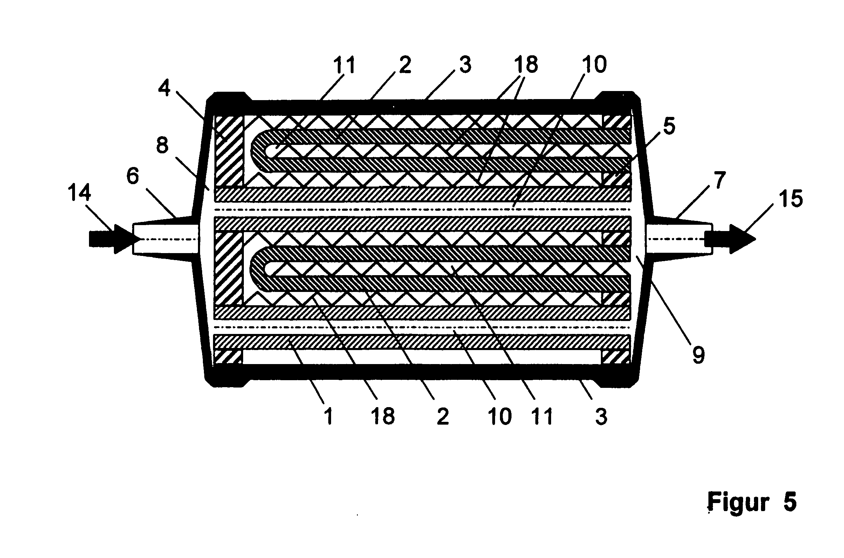

Eine Ausführungsform des erfindungsgemäßen Membranmoduls, bei dem die zweiten Membranelemente aus Flachmembranen ausgebildet sind, ist in Figur 5 dargestellt. Der Modul gemäß Figur 5 weist eine Anordnung aus ersten Lagen mit ersten Membranelementen 1 in Form von Hohlfasermembranen und zweiten Lagen mit zweiten Membranelementen 2 auf, die aus U-förmig gefalteten Flachmembranen gebildet sind. Der Hohlraum 11 der zweiten Membranelemente wird gemäß der Schnittdarstellung durch die bei der U-förmigen Faltung entstehenden Schenkel der jeweiligen Flachmembran sowie durch die U-förmige Faltkante begrenzt und entsprechend dem gezeigten Beispiel in Figur 5 durch einen Abstandshalter 18 stabilisiert. Gleichzeitig sind auch Abstandshalter 18 zwischen die ersten Membranelemente 1 und die zweiten Membranelemente 2 eingebracht, um eine stabile Positionierung insbesondere der zweiten Membranelemente 2 im Modulgehäuse 3 zu erreichen.

Die Faltkanten, welche in Richtung des Verteilerraums 9 angeordnet und im vorliegenden Beispiel nicht zusammen mit den entsprechenden Enden der ersten Membranelemente 1 in die Vergussmasse 4 eingebettet sind, schließen zusammen mit den an die Faltkante angrenzenden, ebenfalls geschlossenen Seitenkanten die aus Flachmembranen ausgebildeten zweite Membranelemente 2 gegenüber dem Außenraum ab, so dass die Hohlräume dieser Elemente an ihrem dem Verteilerraum 8 zugewandten Ende geschlossen sind. Auf diese Weise werden die zweiten Membranelemente 2 in der Anwendung vom Permeat, welches durch die Wände der ersten Membranelemente permeiert ist, im dead-end Modus durchströmt. Zum Sammelraum 9 hin sind die Hohlräume 11 der zweiten Membranelemente 2 geöffnet, so dass das Permeat aus den Hohlräumen der zweiten Membranelemente 2 in den Sammelraum 9 ausströmen kann, wo es mit dem aus den Hohlräumen 10 der ersten Membranelemente 1 ausströmenden Retentat vereinigt wird.

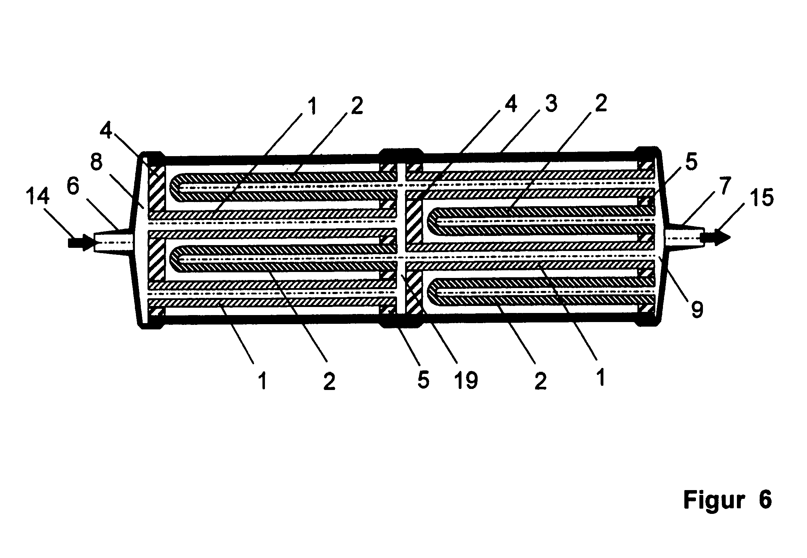

Figur 6 zeigt einen erfindungsgemäßen Membranmodul, der zwei Membranelementeanordnungen aus ersten und zweiten Membranelementen 1,2 als in Richtung der Durchströmung des Gehäuses 3 gesehen hintereinandergeschaltete Modulstufen enthält. Der Aufbau der einzelnen Anordnungen entspricht demjenigen der in Figur 5 gezeigten Anordnung aus ersten und zweiten Membranelementen 1,2 mit Hohlfasermembranen als ersten Membranelementen 1 und U-förmig gefalteten Flachmembranen als zweiten Membranelementen 2. Die einzelnen Modulstufen weisen zueinander einen Abstand auf, so dass zwischen den Modulstufen ein frei durchströmbarer Raum 19 entsteht, der Sammelraum für die, bezogen auf die Durchströmrichtung des Gehäuses, vor dem Raum 19 liegende erste Modulstufe ist und in dem Retentat und Permeat aus der ersten Modulstufe zusammengeführt werden. Gleichzeitig ist dieser Raum 19 Verteilerraum für die, bezogen auf die Durchströmrichtung, hinter dem Raum 19 liegende zweite Modulstufe, aus dem das in der ersten Modulstufe behandelte Fluid auf die ersten Membranelemente 1 der zweiten Modulstufe verteilt wird. Das die zweite Modulstufe verlassende Retentat und Permeat wird im Sammelraum 9 zusammengeführt, und das in zwei Modulstufen behandelte Fluid 15 über die Auslasseinrichtung 7 aus dem Membranmodul abgeleitet.

## Patentansprüche

1. Membranmodul zur stoffspezifischen Behandlung eines Fluids, enthaltend
a) ein Gehäuse (3),
b) eine Einlasseinrichtung (6) zum Einleiten des zu behandelnden Fluids (14) in das Gehäuse (6) in einen Verteilerraum (8),
c) eine Auslasseinrichtung (7) zum Ableiten des behandelten Fluids (15) aus dem Gehäuse (3) aus einem Sammelraum (9), und
d) im Gehäuse (3) angeordnete erste Membranelemente (1) mit poröser semipermeabler Wand sowie jeweils einem dem Verteilerraum (8) und einem dem Sammelraum (9) zugewandten Ende und einem durch die Wand ausgebildeten Hohlraum (10), wobei die ersten Membranelemente (1) mit ihrem dem Verteilerraum (8) zugewandten Ende in eine erste Vergussmasse (4) und mit ihrem dem Sammelraum (9) zugewandten Ende in eine zweite Vergussmasse (5) so eingebettet sind, dass die Enden durch die Vergussmassen (4,5) hindurchtreten und die Hohlräume (10) der ersten Membranelemente (1) jeweils sowohl an dem dem Verteilerraum (8) zugewandten Ende als auch an dem dem Sammelraum (9) zugewandten Ende offen ist und in den Verteilerraum (8) und in den Sammelraum (9) münden,
**dadurch gekennzeichnet, dass** der Membranmodul des Weiteren im Gehäuse (3) angeordnete zweite Membranelemente (2) mit poröser semipermeabler Wand sowie jeweils einem dem Verteilerraum (8) und einem dem Sammelraum (9) zugewandten Ende und einem durch die Wand ausgebildeten Hohlraum (11) enthält, wobei die zweiten Membranelemente (2) mit ihren dem Sammelraum (9) zugewandten Enden zusammen mit den ersten Membranelementen (1) in die zweite Vergussmasse (5) so eingebettet sind, dass diese Enden durch die zweite Vergussmasse (5) hindurchtreten und die Hohlräume (11) der zweiten Membranelemente (2) an diesen Enden jeweils offen sind und in den Sammelraum münden, und die zweiten Membranelemente (2) an ihren dem Verteilerraum (8) zugewandten Enden geschlossen sind, wobei die ersten und die zweiten Membranelemente (1,2) eine Membranelementeanordnung ausbilden und wobei zwischen den Vergussmassen (4,5) ein gegenüber dem Verteilerraum (8) und gegenüber dem Sammelraum (9) fluiddicht abgetrennter und durch die Vergussmassen (4,5), die Innenwand des Gehäuses (3) und die Membranelemente (1,2) begrenzter Außenraum (12) ausgebildet ist.

2. Membranmodul nach Anspruch 1, **dadurch gekennzeichnet, dass** die Membranelementeanordnung aus flächigen Schichten aufgebaut ist.

3. Membranmodul nach Anspruch 2, **dadurch gekennzeichnet, dass** die Membranelementeanordnung aus ebenen flächigen Schichten aufgebaut ist, die zu einem Stapel aufeinandergelegt sind.

4. Membranmodul nach Anspruch 2, **dadurch gekennzeichnet, dass** die Membranelementeanordnung aus flächigen Schichten besteht, die spiralförmig um eine Wickelachse gewickelt sind.

5. Membranmodul nach einem oder mehreren der Ansprüche 2 bis 4, **dadurch gekennzeichnet, dass** die Membranelementeanordnung erste Schichten und zweite Schichten umfasst, wobei die ersten Schichten nur erste Membranelemente (1) und die zweiten Schichten nur zweite Membranelemente (2) enthalten.

6. Membranmodul nach Anspruch 5, **dadurch gekennzeichnet, dass** die Membranelementeanordnung eine alternierende Abfolge von ersten und zweiten Schichten umfasst.

7. Membranmodul nach einem oder mehreren der Ansprüche 1 bis 6, **dadurch gekennzeichnet, dass** die ersten Membranelemente (1) und die zweiten Membranelemente (2) Hohlfasermembranen sind.

8. Membranmodul nach einem oder mehreren der Ansprüche 2 bis 6, **dadurch gekennzeichnet, dass** die ersten Membranelemente (1) und zweiten Membranelemente (2) Hohlfasermembranen sind und die Hohlfasermembranen innerhalb der Schichten zueinander im wesentlichen parallel angeordnet sind.

9. Membranmodul nach einem oder mehreren der Ansprüche 7 oder 8, **dadurch gekennzeichnet, dass** die Hohlfasermembranen in mindestens eine Hohlfasermatte eingebunden sind.

10. Membranmodul nach Anspruch 9, **dadurch gekennzeichnet, dass** die Hohlfasermembranen mittels textiler Fäden in die mindestens eine Hohlfasermatte eingebunden sind.

11. Membranmodul nach einem oder mehreren der Ansprüche 9 oder 10, **dadurch gekennzeichnet, dass** die mindestens eine Hohlfasermatte zick-zack-förmig zu einem Stapel gefaltet ist, der die Membranelementeanordnung ausbildet.

12. Membranmodul nach einem oder mehreren der Ansprüche 1 bis 6, **dadurch gekennzeichnet, dass** die ersten Membranelemente (1) Hohlfasermembranen sind und die zweiten Membranelemente (2) jeweils aus mindestens einer Flachmembran ausgebildet sind.

13. Membranmodul nach Anspruch 12, **dadurch gekennzeichnet, dass** die zweiten Membranelemente (2) jeweils aus einer U-förmig gefalteten, im wesentlichen rechteckigen oder quadratischen Flachmembran gebildet sind, wobei neben der Faltkante zwei weitere Seitenkanten geschlossen sind, die durch die Faltung entstandenen Schenkel der U-förmig gefalteten Flachmembran zur Bildung des Hohlraums (11) auf Abstand gehalten sind und die verbleibende offene Seitenkante in Richtung des Sammelraums (9) angeordnet ist.

14. Membranmodul nach Anspruch 12, **dadurch gekennzeichnet, dass** die zweiten Membranelemente (2) aus zwei zueinander im wesentlichen parallelen und im wesentlichen rechteckigen oder quadratischen Flachmembranen ausgebildet sind, die so angeordnet sind, dass ihre Kanten parallel zueinander verlaufen, wobei die Flachmembranen zueinander auf Abstand gehalten und an ihrer in Richtung Verteilerraum (8) weisenden Kante sowie den dazu senkrechten Kanten formschlüssig miteinander verbunden sind und die verbleibende offene Seitenkante in Richtung des Sammelraums (9) angeordnet ist.

15. Membranmodul nach einem oder mehreren der Ansprüche 1 bis 14, **dadurch gekennzeichnet, dass** im Gehäuse (3) eine Matrix enthalten ist, auf und/oder in der funktionelle Gruppen immobilisiert sind.

16. Membranmodul nach Anspruch 15, **dadurch gekennzeichnet, dass** die ersten und/oder die zweiten Membranelemente (1,2) Matrix für die funktionellen Gruppen sind.

17. Membranmodul nach einem oder mehreren der Ansprüche 15 oder 16, **dadurch gekennzeichnet, dass** zwischen die ersten und zweiten Membranelemente fluiddurchlässige Trägermaterialien eingebracht sind, die Matrix für die funktionellen Gruppen sind.

18. Membranmodul nach Anspruch 17, **dadurch gekennzeichnet, dass** die Trägermaterialien semipermeable poröse Flachmembranen (16) sind.

19. Membranmodul nach Anspruch 17, **dadurch gekennzeichnet, dass** die Trägermaterialien Partikel (17) sind.

20. Membranmodul nach einem oder mehreren der Ansprüche 17 bis 21, **dadurch gekennzeichnet, dass** die Matrix unterschiedliche funktionelle Gruppen aufweist.

21. Membranmodul nach einem oder mehreren der Ansprüche 1 bis 20, **dadurch gekennzeichnet, dass** in Richtung der Erstreckung des Gehäuses (3) zwischen Einlasseinrichtung (6) und Auslasseinrichtung (7) des Membranmoduls mehrere Membranelementeanordnungen räumlich hintereinander als Stufen angeordnet sind.

22. Verfahren zur stoffspezifischen Behandlung eines Fluids, welches zumindest die Schritte umfasst
a) Einleiten des zu behandelnden Fluids (14) als Speisestrom in einen mindestens eine erste Membran (1) und mindestens eine zweite Membran (2) enthaltenden Modul,
b) Vorbeileiten des Speisestroms im cross-flow Modus an der einen Seite der ersten Membran (1), so dass ein Teil des Speisestroms als Permeat durch die erste Membran (1) hindurchtritt und auf der anderen Seite der ersten Membran (1) aus dieser austritt, während der Rest des Speisestroms als Retentat entlang der einen Seite der ersten Membran (1) weiterströmt,
c) Einleiten zumindest eines wesentlichen Teils des Permeats im dead-end Modus in die zweite Membran (2) auf der einen Seite dieser Membran (2), Durchströmen der zweiten Membran (2) und Ausleiten aus der zweiten Membran (2) auf deren anderer Seite,
d) Vereinigung des Permeats mit dem Retentat zum behandelten Fluid innerhalb des Moduls und
e) Ableiten des behandelten Fluids (15) aus dem Modul,
wobei das Permeat zwischen der Abtrennung vom Speisestrom und der Vereinigung mit dem Retentat mindestens einer stoffspezifischen Behandlung unterworfen wird.

23. Verfahren nach Anspruch 22, **dadurch gekennzeichnet, dass** die Schritte b) bis d) mehrfach durchlaufen werden.

24. Verfahren nach einem der Ansprüche 22 oder 23, **dadurch gekennzeichnet, dass** ein Membranmodul gemäß Anspruch 1 eingesetzt wird.

25. Verfahren nach Anspruch 24, **dadurch gekennzeichnet, dass** die Behandlung des Fluids in mehreren in Richtung der Erstreckung des Gehäuses (1) zwischen Einlasseinrichtung (6) und Auslasseinrichtung (7) des Membranmoduls räumlich als Stufen hintereinander angeordneten Membranelementeanordnungen durchgeführt wird.

26. Verfahren nach Anspruch 25, **dadurch gekennzeichnet, dass** die Behandlung in bis zu 10 Stufen durchgeführt wird.

27. Verfahren nach einem oder mehreren der Ansprüche 22 bis 26, **dadurch gekennzeichnet, dass** das behandelte Fluid rezirkuliert wird.

28. Verfahren nach einem oder mehreren der Ansprüche 22 bis 27, **dadurch gekennzeichnet, dass** das zu behandelnde Fluid (14) eine Suspension ist.

29. Verfahren nach einem oder mehreren der Ansprüche 22 bis 28, **dadurch gekennzeichnet, dass** das Permeat zwischen der Abtrennung vom Speisestrom und der Vereinigung mit dem Retentat eine Matrix durchströmt, auf und/oder in der auf die stoffspezifische Behandlung abgestimmte funktionelle Gruppen immobilisiert sind.

30. Verfahren zur Reinigung/Trennung von Ligaten aus einer ligathaltigen Flüssigkeit mittels Affinität, **dadurch gekennzeichnet, dass** eine Matrix ausgewählt wird, auf und/oder in der Liganden für besagte Ligaten immobilisiert sind, und das Verfahren gemäß Anspruch 29 durchgeführt wird.

31. Verfahren zur katalytischen Behandlung von Fluiden, **dadurch gekennzeichnet, dass** eine Matrix ausgewählt wird, auf und/oder in der Katalysatoren immobilisiert sind, und das Verfahren gemäß Anspruch 29 durchgeführt wird.

## Claims

1. Membrane module for substance-specific treatment of a fluid, comprising
a) a housing (3),
b) an inlet arrangement (6) for introducing the fluid (14) to be treated into a distribution space (8) in the housing (3),
c) an outlet arrangement (7) for discharging the treated fluid (15) from a collection space (9) in the housing (3),
d) first membrane elements (1) arranged in the housing (3) with porous semi-permeable wall, each with one end pointing towards the distribution space (8) and one end pointing towards the collection space (9) and a cavity (10) formed by the wall, whereby the first membrane elements (1) with their end pointing towards the distribution space (8) are embedded in a first sealing compound (4) and with their end pointing towards the collection space (9) are embedded in a second sealing compound (5) in such a way that the ends protrude through the sealing compounds (4,5) and the cavities (10) of the first membrane elements (1) are each open at both the end facing towards the collection space (9) and at the end facing towards the distribution space (8) and terminate in the distribution space (8) and the collection space (9),
**characterised in that** the membrane module furthermore comprises second membrane elements (2) arranged in the housing (3) with porous semi-permeable wall, each with one end pointing towards the distribution space (8) and one end pointing towards the collection space (9) and a cavity (11) formed by the wall, whereby the second membrane elements (2) with their end pointing towards the collection space (9) are embedded together with the first membrane elements (1) in the second sealing compound (5) in such a way that these ends protrude through the second sealing compound (5) and the cavities (11) of the second membrane elements (2) are each open at these ends and terminate in the collection space (9), and the second membrane elements (2) are closed at their ends facing towards the distribution space (8), with the first and the second membrane elements (1,2) forming a membrane element arrangement, and with an outer space (12) being formed between the sealing compounds (4,5) that is delimited by the sealing compounds (4,5), the inner wall of the housing (3) and the membrane elements (1,2) and that is separated fluid-tight from the distribution space (8) and from the collection space (9).

2. Membrane module according to Claim 1, **characterised in that** the membrane element arrangement is made up of flat layers.

3. Membrane module according to Claim 2, **characterised in that** the membrane element arrangement is made up of plane flat layers that are placed onto one another to form a stack.

4. Membrane module according to Claim 2, **characterised in that** the membrane element arrangement is made up of flat layers that are wound spirally around a winding axis.

5. Membrane module according to one or more of Claims 2 to 4, **characterised in that** the membrane element arrangement comprises first layers and second layers, with the first layers containing only first membrane elements (1) and the second layers containing only second membrane elements (2).

6. Membrane module according to Claim 5, **characterised in that** the membrane element arrangement comprises an alternating sequence of first and seconds layers.

7. Membrane module according to one or more of Claims 1 to 6, **characterised in that** the first membrane elements (1) and the second membrane elements (2) are hollow-fibre membranes.

8. Membrane module according to one or more of Claims 2 to 6, **characterised in that** the first membrane elements (1) and the second membrane elements (2) are hollow-fibre membranes and the hollow-fibre membranes within the layers are arranged essentially parallel to one another.

9. Membrane module according to one or more of Claims 7 or 8, **characterised in that** the hollow-fibre membranes are bound into at least one hollow-fibre mat.

10. Membrane module according to Claim 9, **characterised in that** the hollow-fibre membranes are bound by means of textile threads into the at the least one hollow-fibre mat.

11. Membrane module according to one or more of Claims 9 or 10, **characterised in that** the at least one hollow-fibre mat is folded in a zigzag form into a stack that forms the membrane element arrangement.

12. Membrane module according to one or more of Claims 1 to 6, **characterised in that** the first membrane elements (1) are hollow-fibre membranes and the second membrane elements (2) are each formed from at least one flat membrane.

13. Membrane module according to Claim 12, **characterised in that** the second membrane elements (2) are each made of an essentially square or rectangular flat membrane folded in a U-shape, whereby in addition to the fold edge two further side edges are closed, the flanks, formed by folding, of the flat membrane folded in a U-shape are held at a distance from one another to form the cavity (11) and the remaining open side edge is arranged towards the collection space (9).

14. Membrane module according to Claim 12, **characterised in that** the second membrane elements (2) are each made of two essentially square or rectangular flat membranes arranged essentially parallel to one another that are arranged in such a way that their edges run parallel to one another, whereby the flat membranes are held at a distance from one another and at their edge facing towards the distribution space (8) and the perpendicular edges thereto are positively joined and the remaining open side edge is arranged towards the collection space (9).

15. Membrane module according to one or more of Claims 1 to 14, **characterised in that** a matrix on and/or in which functional groups are immobilised is contained in the housing (3).

16. Membrane module according to Claim 15, **characterised in that** the first and/or the second membrane elements (1,2) are the matrix for the functional groups.

17. Membrane module according to one or more of Claims 15 or 16, **characterised in that** fluid-permeable carrier materials are arranged between the first and second membrane elements that are the matrix for the functional groups.

18. Membrane module according to Claim 17, **characterised in that** the fluid-permeable carrier materials are semi-permeable porous flat membranes (16).

19. Membrane module according to Claim 17, **characterised in that** the carrier materials are particles (17).

20. Membrane module according to one or more of Claims 17 to 19, **characterised in that** the matrix has different functional groups.

21. Membrane module according to one or more of Claims 1 to 20, **characterised in that** several membrane element arrangements are arranged spatially in series as stages in the direction of the extent of the housing (3) of the membrane module between the inlet arrangement (6) and outlet arrangement (7).

22. Process for substance-specific treatment of a fluid comprising at least the steps
a) Introducing the fluid (14) to be treated as a feed stream into a module containing at least one first membrane (1) and at least one second membrane (2),
b) Bypassing the feed stream in cross-flow mode on one side of the first membrane (1) so that a portion of the feed stream passes as permeate through the first membrane (1) and leaves the same on the other side of the first membrane (1), while the rest of the feed stream continues to flow along the one side of the first membrane (1) as a retentate,
c) Introducing at least a significant portion of the permeate in dead-end mode into the second membrane (2) on the one side of this membrane (2), flowing through the second membrane (2) and discharging out of the second membrane (2) on its other side,
d) Uniting the permeate with the retentate to the treated fluid within the module, and
e) Discharging the treated fluid (15) out of the module, whereby the permeate is subjected to least one substance-specific treatment between the separation from the feed stream and the unification with the retentate.

23. Process according to Claim 22, **characterised in that** steps b) to d) are performed several times.

24. Process according to one of Claims 22 or 23, **characterised in that** a membrane module according to Claim 1 is employed.

25. Process according to Claim 24, **characterised in that** the treatment of the fluid is performed in several membrane element arrangements arranged spatially in series as stages in the direction of the extent of the housing (3) of the membrane module between the inlet arrangement (6) and outlet arrangement (7).

26. Process according to Claim 25, **characterised in that** the treatment is performed in up to 10 stages.

27. Process according to one or more of Claims 22 to 26, **characterised in that** the treated fluid is recirculated.

28. Process according to one or more of Claims 22 to 27, **characterised in that** the fluid (14) to be treated is a suspension.

29. Process according to one or more of Claims 22 to 28, **characterised in that** between the separation from the feed stream and the unification with the retentate, the permeate flows through a matrix on and/or in which functional groups matched to the substance-specific treatment are immobilised.

30. Process for cleaning/separation of ligates from a ligate-containing liquid by affinity, **characterised in that** a matrix is selected on and/or in which ligands for said ligates are immobilized, and that the process is conducted according to Claim 29.

31. Process for catalytic treatment of fluids, **characterised in that** a matrix is selected on and/or in which catalysts are immobilized, and that the process is conducted according to Claim 29.

## Revendications

1. Module à éléments de membrane pour le traitement spécifique de substance d'un fluide, contenant
a) un logement (3),
b) un dispositif d'entrée (6) pour l'introduction du fluide à traiter (14) dans le logement (6) dans un espace de distribution (8),
c) un dispositif de sortie (7) pour l'évacuation du fluide traité (15) hors du logement (3) depuis un espace de collecte (9), et
d) des premiers éléments de membrane (1) agencés dans le logement (3), avec paroi poreuse semi-perméable, ainsi que chaque fois, une extrémité orientée vers l'espace de distribution (8) et l'espace de collecte (9), et un espace vide (10) formé par la paroi, où les premiers éléments de membrane (1) sont insérés dans une première masse de scellement (4) par leurs extrémités orientées vers l'espace de distribution (8) et dans une deuxième masse de scellement (5) par leurs extrémités orientées vers l'espace de collecte (9), de sorte que les extrémités traversent les masses de scellement (4, 5) et que les espaces vides (10) des premiers éléments de membrane (1) sont ouverts chaque fois sur l'extrémité orientée vers l'espace de distribution (8), mais également sur l'extrémité orientée vers l'espace de collecte (9) et débouche dans l'espace de distribution (8) et dans l'espace de collecte (9),
**caractérisé en ce que** le module de membrane contient par ailleurs, des deuxièmes éléments de membrane (2) agencés dans le logement (3), avec paroi poreuse semi-perméable, ainsi que chaque fois, une extrémité orientée vers l'espace de distribution (8) et l'espace de collecte (9), et un espace vide (11) formé par la paroi, où les deuxièmes éléments de membrane (2) sont insérés dans la deuxième masse de scellement (5) par leurs extrémités orientées vers l'espace de collecte (9), avec les premiers éléments de membrane (1) de sorte que ces extrémités traversent la deuxième masse de scellement (5) et que les espaces vides (11) des deuxièmes éléments de membrane (2) sont ouverts chaque fois sur ces extrémités et débouchent dans l'espace collecte, et les deuxièmes éléments de membrane (2) sont fermés sur leur extrémité orientée vers l'espace de distribution (8), où les premiers et deuxièmes éléments de membrane (1, 2) forment un agencement d'éléments de membrane et où entre les masses de scellement (4, 5), est formé un espace extérieur (12) séparé de manière étanche aux fluides de l'espace de distribution (8) et de l'espace de collecte (9) et limité par les masses de scellement (4, 5), la paroi intérieure du logement (3) et les éléments de membrane (1, 2).

2. Module à éléments de membrane selon la revendication 1, **caractérisé en ce que** l'agencement des éléments de membrane est élaboré à partir de couches planaires.

3. Module à éléments de membrane selon la revendication 2, **caractérisé en ce que** l'agencement des éléments de membrane est élaboré à partir de couches planaires, qui sont disposées en un empilement.

4. Module à éléments de membrane selon la revendication 2, **caractérisé en ce que** l'agencement des éléments de membrane est élaboré à partir de couches planaires, qui sont enroulées en spirale autour d'un axe d'enroulement.

5. Module à éléments de membrane selon une ou plusieurs des revendications 2 à 4, **caractérisé en ce que** l'agencement des éléments de membrane comprend des premières couches et des deuxièmes couches, où les premières couches ne contiennent que les premiers éléments de membrane (1) et les deuxièmes couches ne contiennent que les deuxièmes éléments de membrane (2).

6. Module à éléments de membrane selon la revendication 5, **caractérisé en ce que** l'agencement des éléments de membrane comprend une succession alternée des premières et deuxièmes couches.

7. Module à éléments de membrane selon une ou plusieurs des revendications 1 à 6, **caractérisé en ce que** les premiers éléments de membrane (1) et les deuxièmes éléments de membrane (2) sont des membranes à fibre creuse.

8. Module à éléments de membrane selon une ou plusieurs des revendications 2 à 6, **caractérisé en ce que** les premiers éléments de membrane (1) et les deuxièmes éléments de membrane (2) sont des membranes à fibre creuse et que les membranes à fibre creuse sont agencées essentiellement parallèlement au sein des couches.

9. Module à éléments de membrane selon une ou plusieurs des revendications 7 ou 8, **caractérisé en ce que** les membranes à fibre creuse sont intégrées en au moins un mat de fibres creuses.

10. Module à éléments de membrane selon la revendication 9, **caractérisé en ce que** les membranes à fibre creuse sont intégrées à l'aide de fils textiles dans le au moins un mat de fibres creuses.

11. Module à éléments de membrane selon une ou plusieurs des revendications 9 ou 10, **caractérisé en ce que** le au moins un mat de fibres creuses est plissé sous forme de zig-zag en un empilement, qui forme l'agencement des éléments de membrane.

12. Module à éléments de membrane selon une ou plusieurs des revendications 1 à 6, **caractérisé en ce que** les premiers éléments de membrane (1) sont des membranes à fibre creuse et les deuxièmes éléments de membrane (2) sont formés chaque fois, par au moins une membrane plane.

13. Module à éléments de membrane selon la revendication 12, **caractérisé en ce que** les deuxièmes éléments de membrane (2) sont formés d'une membrane plane essentiellement rectangulaire ou quadratique, chaque fois pliée en U, où outre le bord de pli, deux autres bords latéraux sont fermés, où les branches formées par pliage de la membrane pliée en U sont maintenues à distance pour former l'espace vide (11), et où le bord latéral ouvert restant est agencé en direction de l'espace de collecte (9).

14. Module à éléments de membrane selon la revendication 12, **caractérisé en ce que** les deuxièmes éléments de membrane (2) sont formés de deux membranes planes essentiellement rectangulaires ou quadratiques et essentiellement parallèles l'une par rapport à l'autre, qui sont agencées de sorte que leurs bords se développent parallèlement, où les membranes planes sont maintenues à distance l'une de l'autre et sont reliées l'une à l'autre par engagement positif, l'une à l'autre sur leur bord en direction de l'espace de distribution (8), ainsi que les bords perpendiculaires à celui-ci, et le bord latéral ouvert restant est agencé en direction de l'espace de collecte (9).

15. Module à éléments de membrane selon une ou plusieurs des revendications 1 à 14, **caractérisé en ce que** dans le logement (3), une matrice est présente, sur et/ou dans laquelle sont immobilisés des groupes fonctionnels.

16. Module à éléments de membrane selon la revendication 15, **caractérisé en ce que** les premiers et/ou les deuxièmes éléments de membrane (1, 2) sont les matrices pour les groupes fonctionnels.

17. Module à éléments de membrane selon une ou plusieurs des revendications 15 ou 16, **caractérisé en ce qu'**entre les premiers et deuxièmes éléments de membrane sont incorporés de manière perméable aux fluides, des matériaux support, qui sont les matrices pour les groupes fonctionnels.

18. Module à éléments de membrane selon la revendication 17, **caractérisé en ce que** les matériaux support sont des membranes planes (16) poreuses semi-perméables.

19. Module à éléments de membrane selon la revendication 17, **caractérisé en ce que** les matériaux support sont des particules (17).

20. Module à éléments de membrane selon une ou plusieurs des revendications 17 à 21, **caractérisé en ce que** la matrice présente des groupes fonctionnels différents.

21. Module à éléments de membrane selon une ou plusieurs des revendications 1 à 20, **caractérisé en ce qu'**en direction du prolongement du logement (3), entre le dispositif d'entrée (6) et le dispositif de sortie (7) du module de membrane, plusieurs agencements d'éléments de membrane sont agencés l'un derrière l'autre, comme des paliers.

22. Procédé pour la traitement spécifique de substance d'un fluide, qui comprend au moins les étapes de
a) introduction du fluide à traiter (14) comme un courant d'alimentation, dans un module contenant au moins une première membrane (1) et au moins une deuxième membrane (2),
b) passage du courant d'alimentation en mode partiel sur une face de la première membrane (1), de sorte qu'une partie du courant d'alimentation traverse la première membrane (1) en tant que perméat et sorte de celui-ci, par l'autre face de la première membrane (1), alors que le reste du courant d'alimentation s'écoule en tant que rétentat, le long d'un côté de la première membrane (1),
c) introduction d'au moins une partie importante du perméat en mode total dans la deuxième membrane (2), sur l'une des faces de cette membrane (2), passage de la deuxième membrane (2) et écoulement de la deuxième membrane (2) sur l'autre côté,
d) rassemblement du perméat avec le rétentat en fluide traité, au sein du module, et
e) évacuation du fluide traité (15) hors du module,
où le perméat est soumis à au moins un traitement spécifique de substance entre la séparation du courant d'alimentation et le rassemblement avec le rétentat.

23. Procédé selon la revendication 22, **caractérisé en ce que** les étapes b) à d) sont réalisées plusieurs fois.

24. Procédé selon l'une des revendications 22 ou 23,
**caractérisé en ce qu'**un module à éléments de membrane selon la revendication 1 est mis en oeuvre.

25. Procédé selon la revendication 24, **caractérisé en ce que** le traitement du fluide est réalisé dans plusieurs agencements d'éléments de membrane disposés l'un derrière l'autre en tant que paliers, en direction du prolongement du logement (1) entre le dispositif d'entrée (6) et le dispositif de sortie (7) du module à éléments de membrane.

26. Procédé selon la revendication 25, **caractérisé en ce que** le traitement est réalisé en jusqu'à 10 paliers.

27. Procédé selon une ou plusieurs des revendications 22 à 26, **caractérisé en ce que** le fluide traité est remis en circulation.

28. Procédé selon une ou plusieurs des revendications 22 à 27, **caractérisé en ce que** le fluide à traiter (147) est une suspension.

29. Procédé selon une ou plusieurs des revendications 22 à 28, **caractérisé en ce que** le perméat traverse une matrice entre la séparation du courant d'alimentation et le rassemblement avec le rétentat, matrice sur et/ou dans laquelle sont mobilisés des groupes fonctionnels destinés au traitement spécifique de substance.

30. Procédé de purification/séparation de ligats depuis un liquide contenant des ligats, par affinité,
**caractérisé en ce que** l'on choisit une matrice, sur et/ou dans laquelle des ligands sont immobilisés pour les ligats, et l'on réalise le procédé selon la revendication 29.

31. Procédé de traitement catalytique de fluides,
**caractérisé en ce que** l'on choisit une matrice, sur et/ou dans laquelle les catalyseurs sont immobilisés, et l'on réalise le procédé selon la revendication 29.
